# EUROPEAN PATENT APPLICATION

(11) **EP 2 210 881 A1**
(43) Date of publication of application: **28.07.2010**
(21) Application number: 09075018.3
(22) Date of filing: 14.01.2009
(51) Int. Cl.: C07D 245/02, C07K 2/00

(54) **Derivatives of syringolin A**

(71) Applicant: Max-Planck-Gesellschaft zur Förderung der Wissenschaften e.V., 80539 München (DE)
(72) Inventor: Kaiser, Markus, 58313 Herdecke (DE); Clerc, Jérôme, 44135 Dortmund (DE)
(74) Representative: Arth, Hans-Lothar

(57) **Abstract**

The present invention relates to syringolin A derivatives, the use of the syringolin A derivatives for prophylaxis and treatment of neurodegenerative diseases and proliferative diseases such as cancer, pharmaceutical compositions containing at least one syringolin A derivative as well as to a synthesis for preparing the syringolin A derivatives.

## Description

The present invention relates to syringolin A derivatives, the use of the syringolin A derivatives for prophylaxis and treatment of neurodegenerative diseases and proliferative diseases such as cancer, pharmaceutical compositions containing at least one syringolin A derivative as well as to a synthesis for preparing the syringolin A derivatives.

### Background of the invention

Syringolin A and syringolin B are plant elicitors produced by the plant pathogen Pseudomonas syringae. Both syringolin A and syringolin B are potent irreversible inhibitors of proteasome. Proteasome inhibitors are compounds that block the action of proteasomes, cellular complexes that break down proteins, like the p53 protein. Proteasome inhibitors are known to be suitable pharmaceutically active compounds for the treatment of proliferative diseases such as cancer.

Syringolin A is an unusual secreted peptide consisting of a 12-membered ring formed by the two non-proteinogenic amino acids 5-methyl-4-amino-2-hexenoic acid and 3,4-dehydrolysine. The α-amino group of the latter is connected by a peptide bond to a valine that in turn is linked to a second valine via a urea moiety.

The chemical formula of syringolin A is as follows:

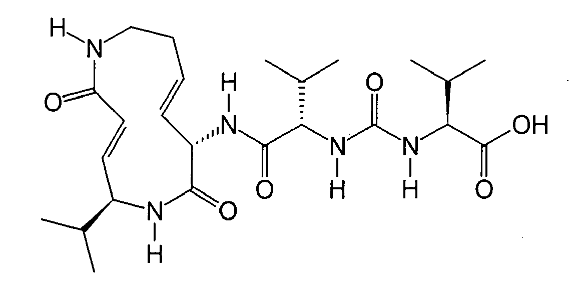

The chemical formula of syringolin B is as follows:

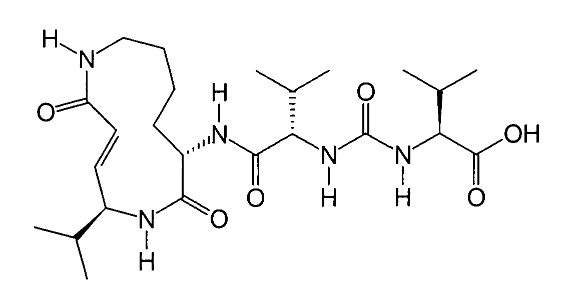

It is the object of the present invention to provide pharmaceutically active derivatives of syringolin A, a synthetic route for preparing pharmaceutically active syringolin A derivatives, pharmaceutical compositions containing at least one syringolin A derivative and the use of such syringolin A derivatives for prophylaxis and treatment of certain diseases.

The object of the present invention is solved by the teaching of the independent claims. Further advantageous features, aspects and details of the invention are evident from the dependent claims, the description, and the examples of the present application.

### Description of the invention

Surprisingly it was found that the syringolin A derivatives of general formula (A) are pharmaceutically highly active compounds. Thus the present invention relates to compounds of general formula (A): 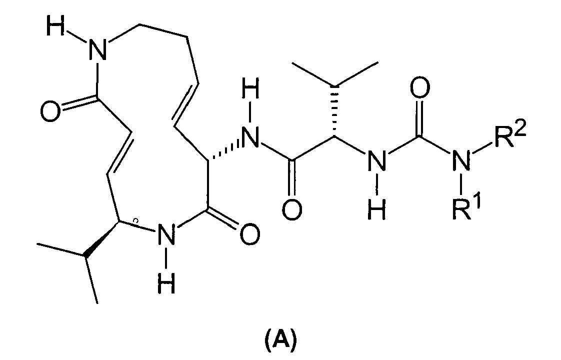 wherein
**R¹** and **R²** represent independently of each other:
   -H, -(CH₂)ₖ-C(R¹²)(R¹³)-(CH₂)ₗ-C(R¹⁴)(R¹⁵)(R¹⁶), -A-R⁴, -A*-R⁵, -A-R⁸, -A*-R⁹, -A-O-R⁸, -A*-O-R⁹, -A-N(R⁸)(R¹⁰), -A*-N(R⁹)(R¹¹), 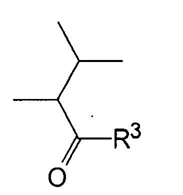 -(CH₂CH₂O)ₘ-R⁸, -(CH₂CH₂O)ₙ-R⁹, -R⁸, -R⁹;
**R³** represents -OH, -O-A-R⁴, -O-A-O-R⁸, -O-A-N(R⁸)(R¹⁰), -(CH₂CH₂O)ₘ-R⁸, -O-R⁸, -NH₂, -NH-A-R⁴, -NH-A-O-R⁸, -NH-A-N(R⁸)(R¹⁰), -NH-R⁸, -N(R⁸)(R¹⁰);
   or the residues -N(**R¹**)(**R²**), -N(**R⁸**)(**R¹⁰**), -N(**R⁹**)(**R¹¹**) represent independently of each other a nitrogen heterocyclic ring selected from: 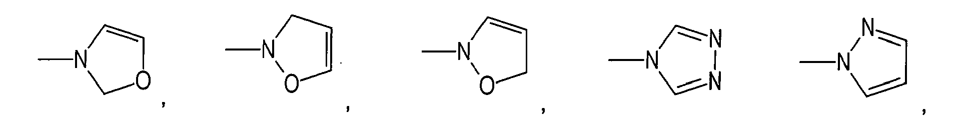 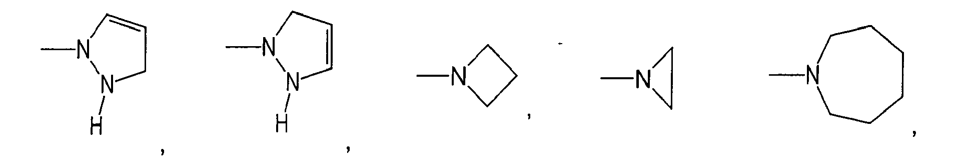 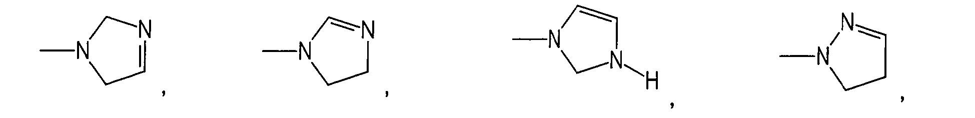 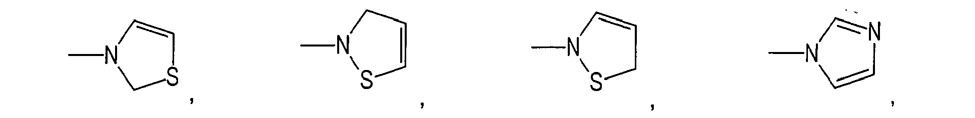 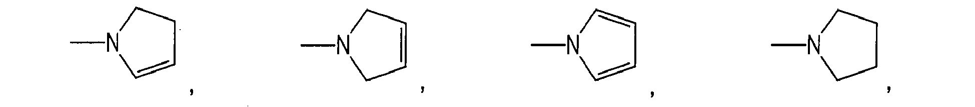 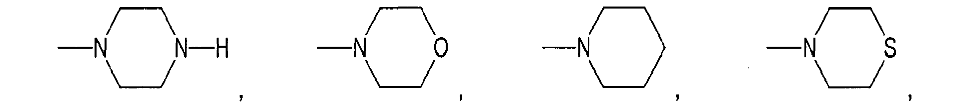 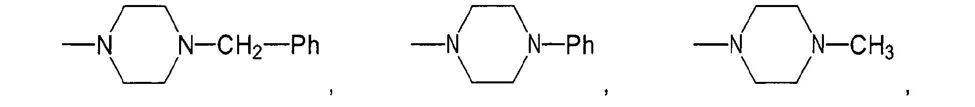 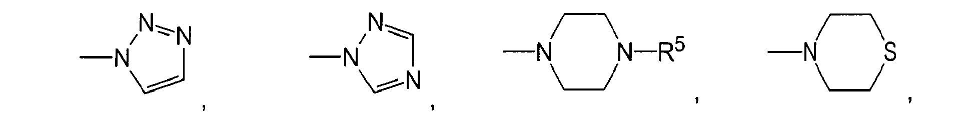 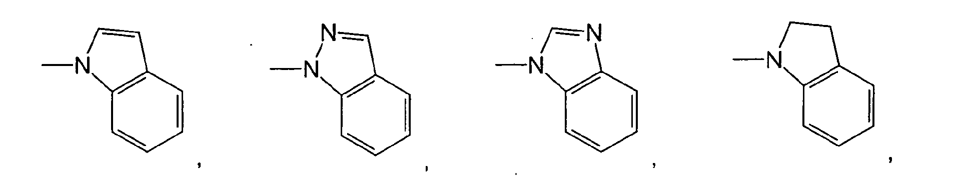 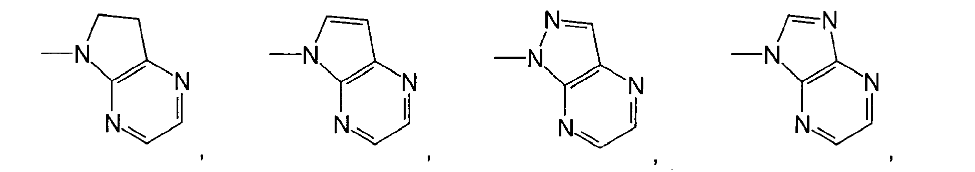 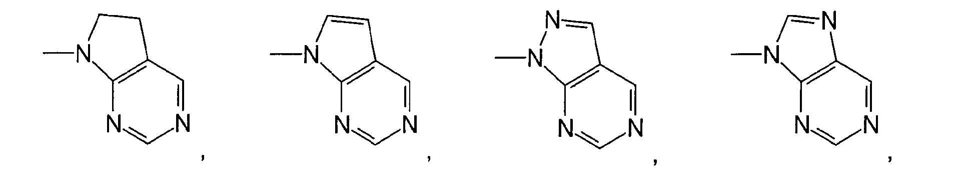 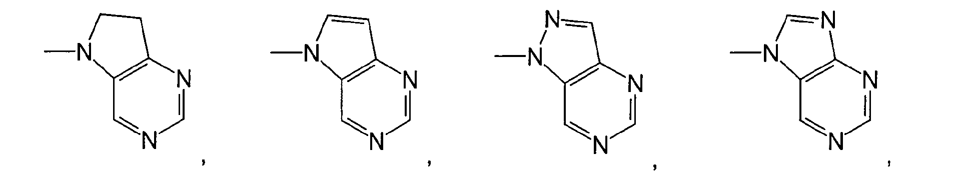 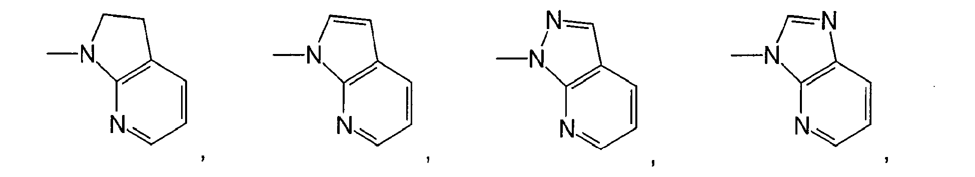 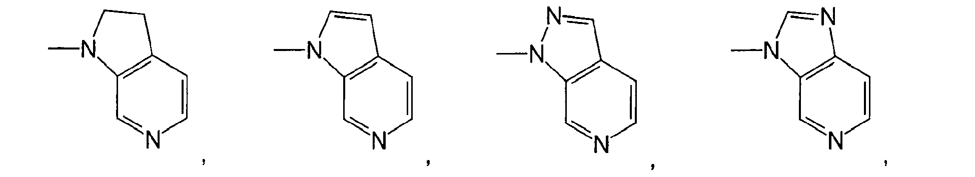 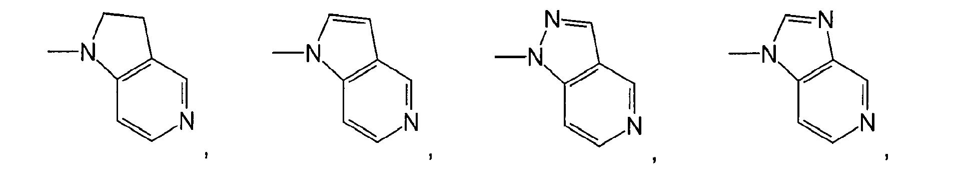 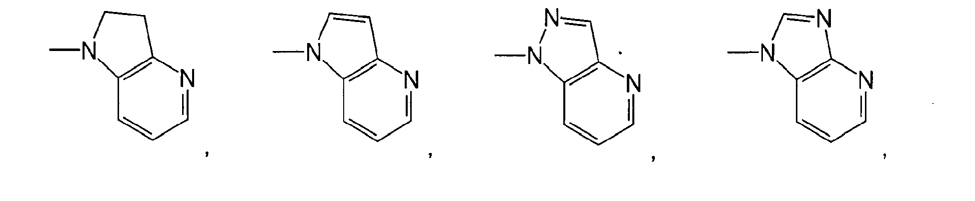 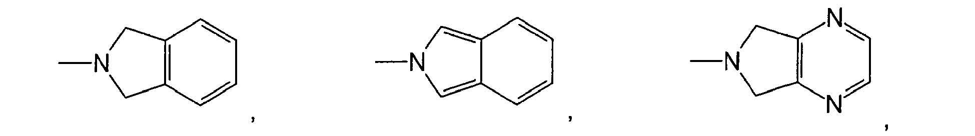 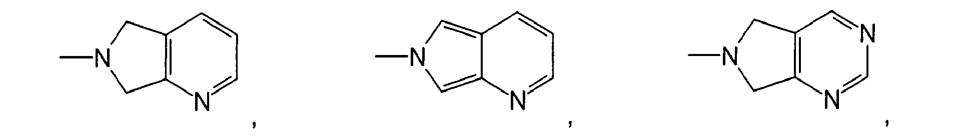 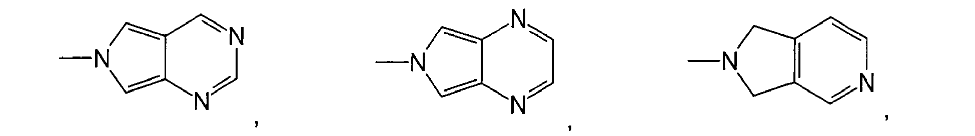 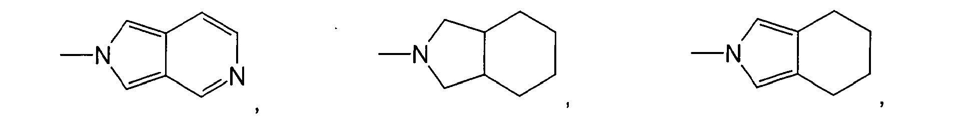 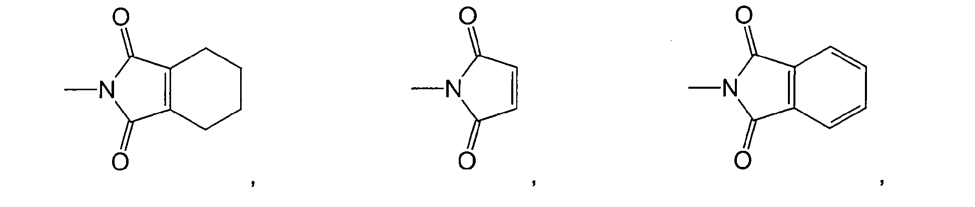 -A- and -A*- represent independently of each other:
   -(CH₂)ₘ-, -(CH₂)ₙ-, -(CH₂)ₚ-(CH=CH)_{q}-(CH₂)ᵣ-, -(CH₂)ᵥ-(o-C₆H₄)_{w}-(CH₂)ₓ-, -(CH₂)ᵥ-(m-C₆H₄)_{w}-(CH₂)ₓ-, -(CH₂)ᵥ-(p-C₆H₄)_{w}-(CH₂)ₓ-, -(CH₂)ₛ-(C≡C)ₜ-(CH₂)ᵤ-;
**R⁴** and **R⁵** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -l, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂₋cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -CON(cyclo-C₃H₅)₂, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)_{2,} -NH-C(=NH)-N(C₃H₇)₂, -NH-C(=NH)-N(cyclo-C₃H₅)₂, -O-CO-NH₂, -NH-C(=NH)-N[CH(CH₃)₂]₂, -NH-C(=NH)-N[C(CH₃)₃]₂, -O-CO-O-cyclo-C₃H₅, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-OCH(CH₃)₂, -O-CO-OC(CH₃)₃; 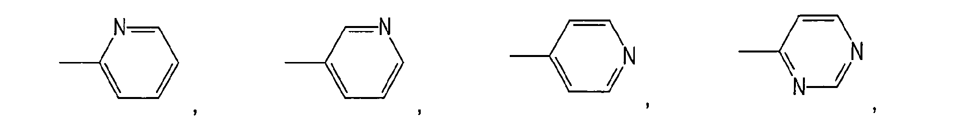 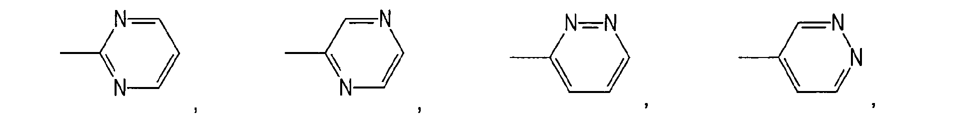 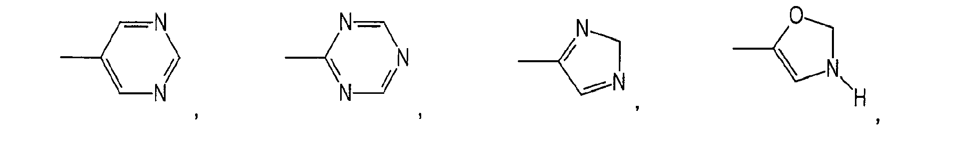 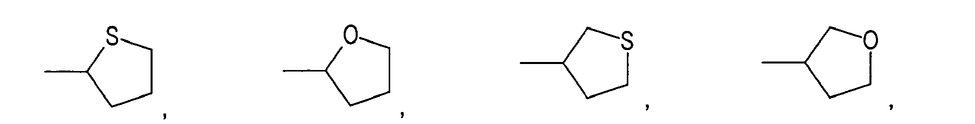 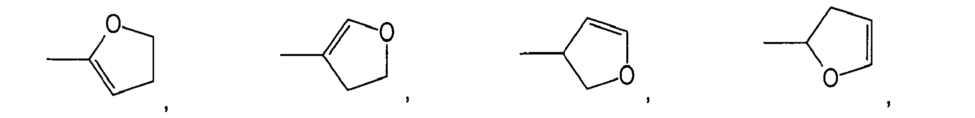 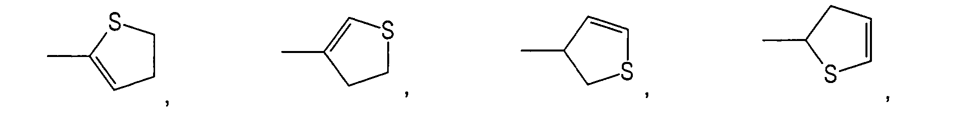 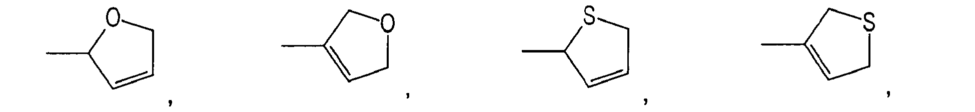 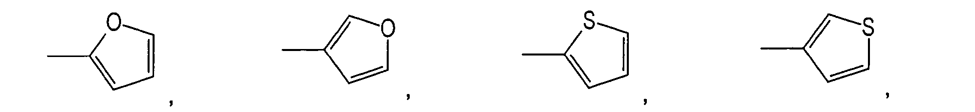 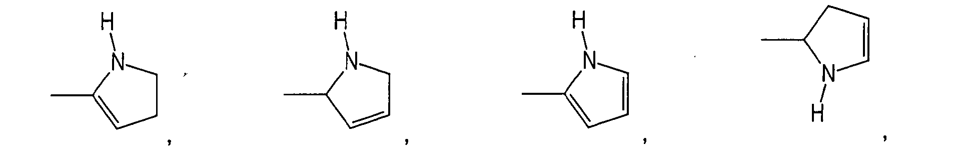 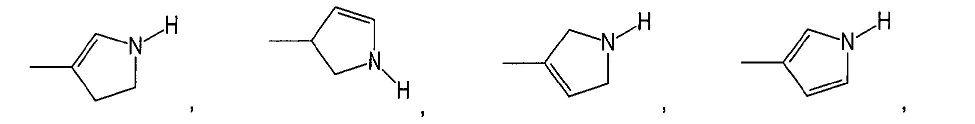 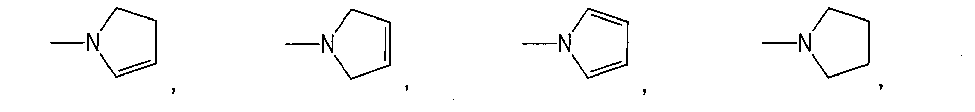 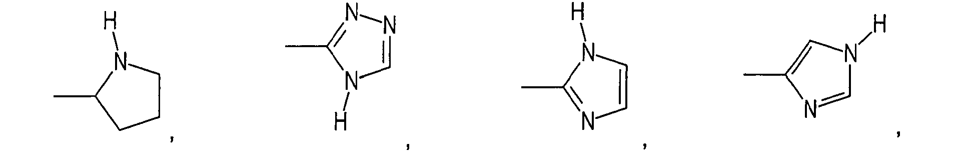 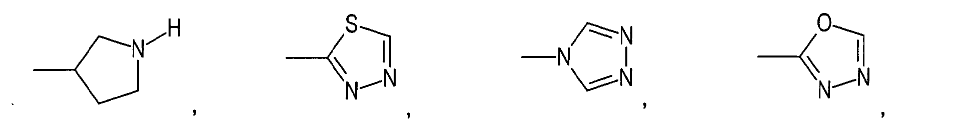 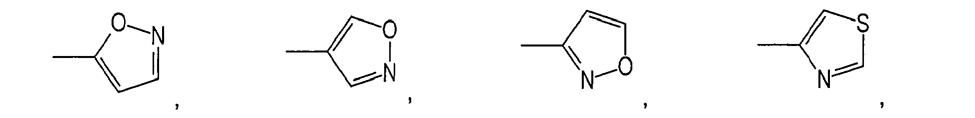 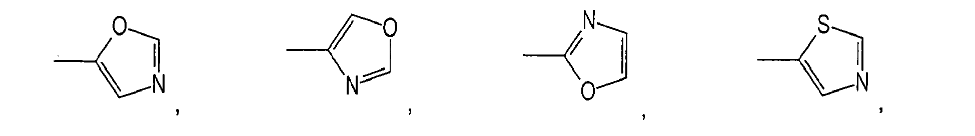 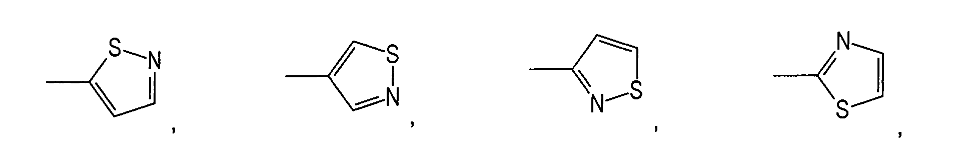 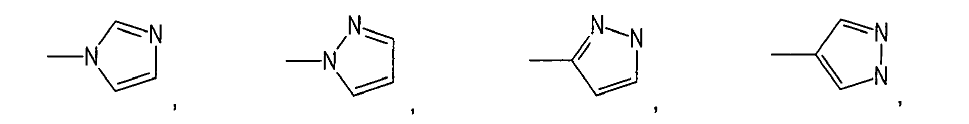 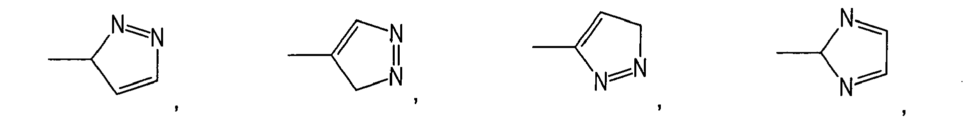 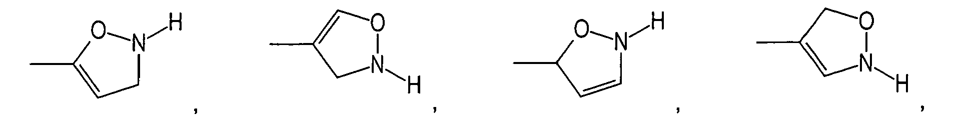 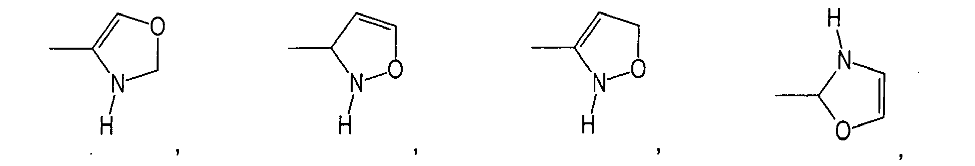 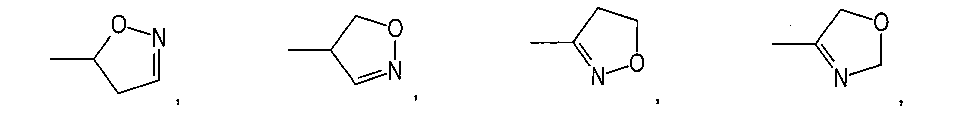 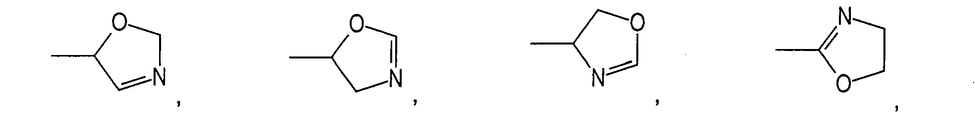 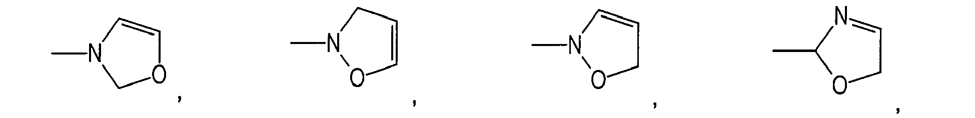 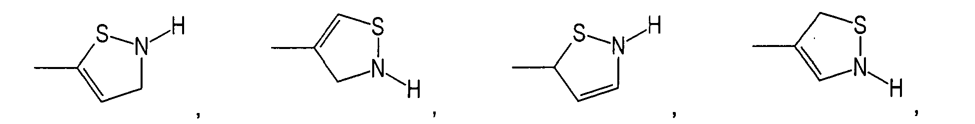 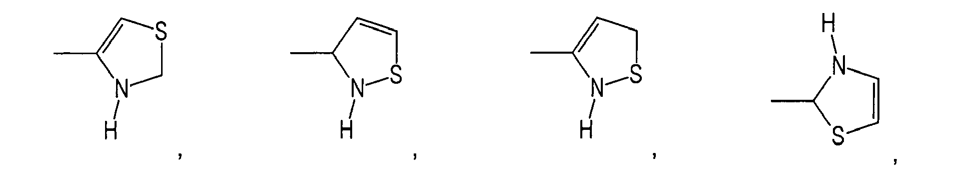 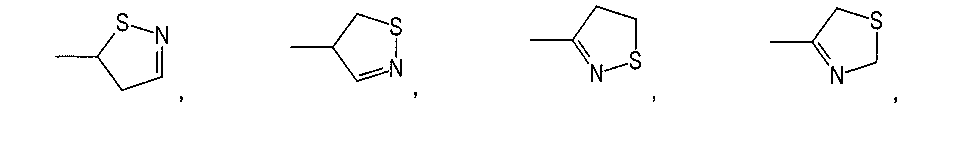 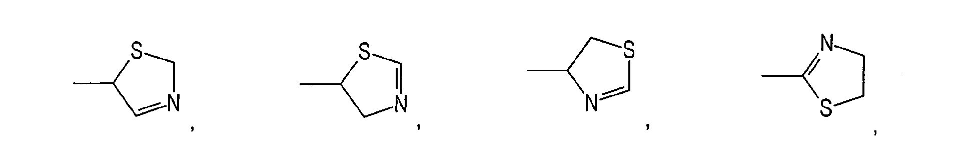 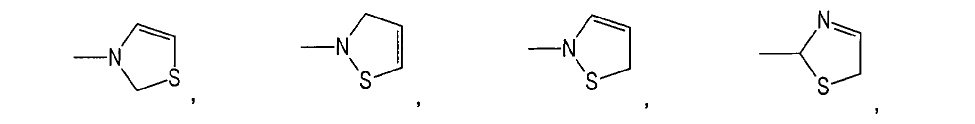 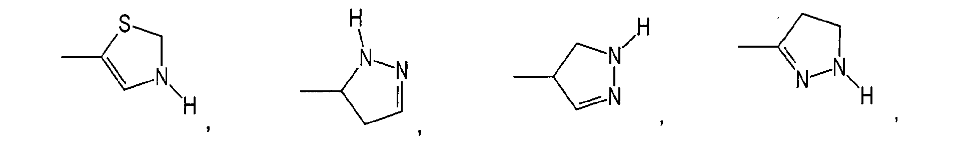 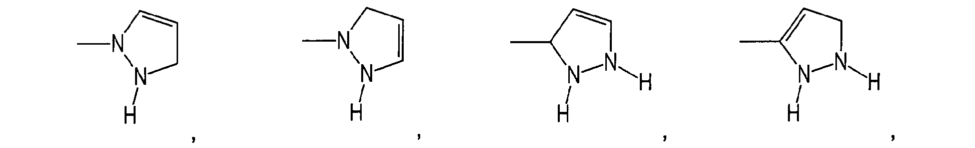 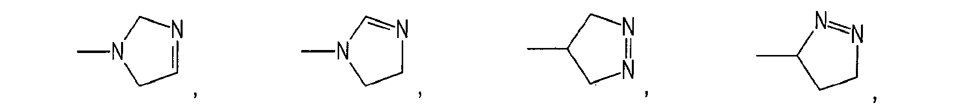 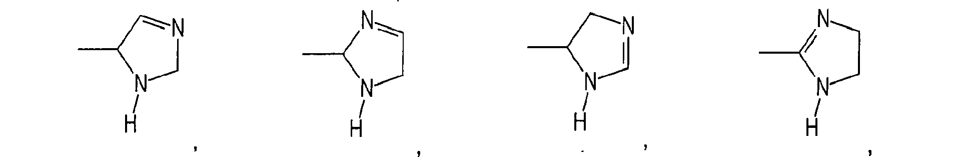 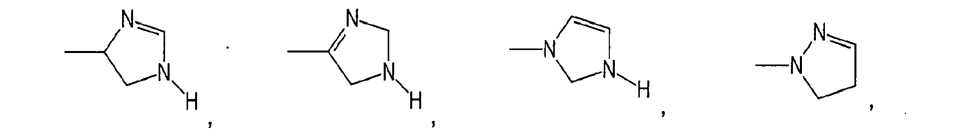 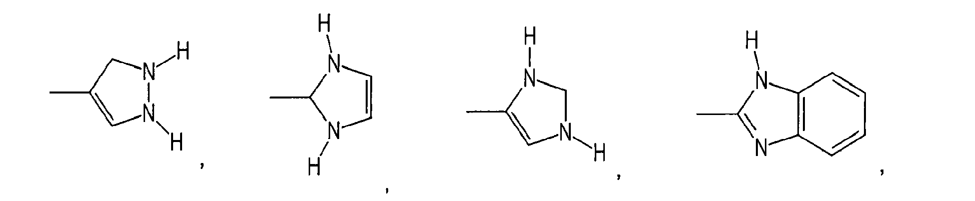 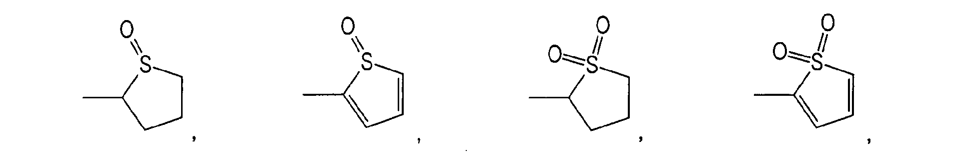 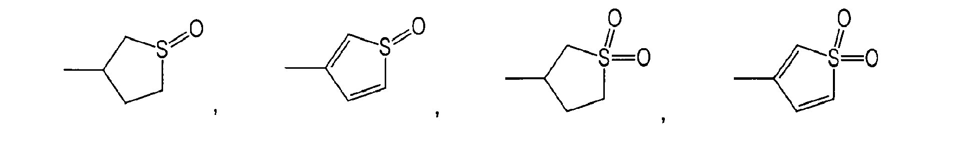 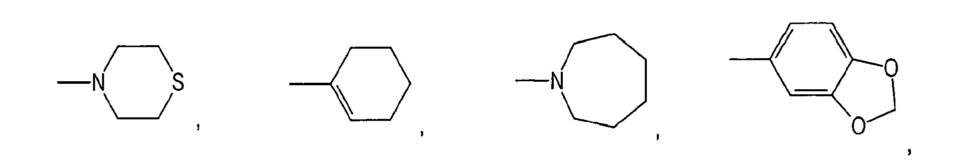 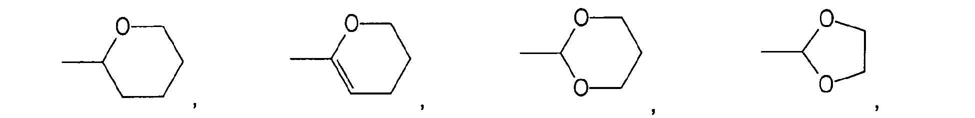 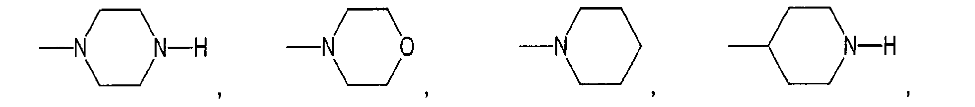 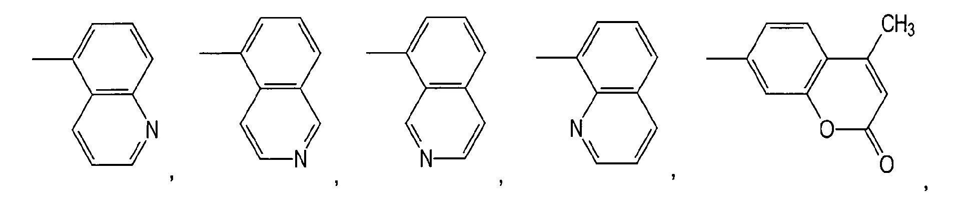 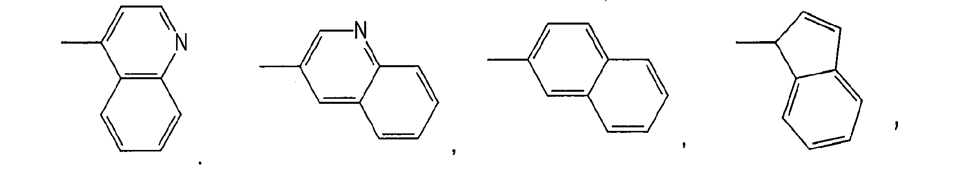 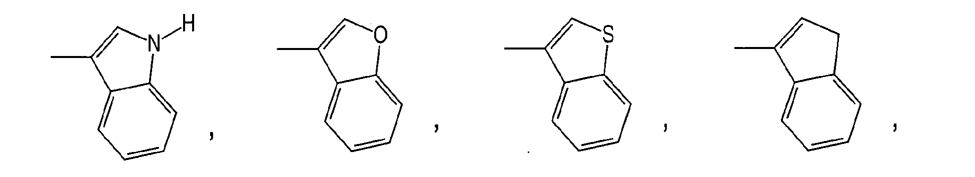 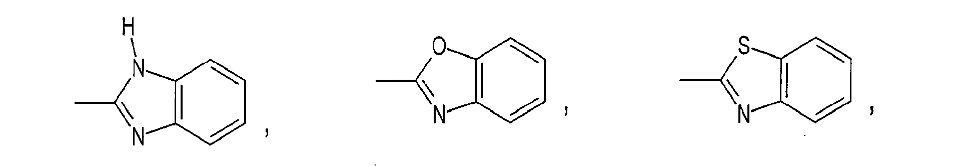 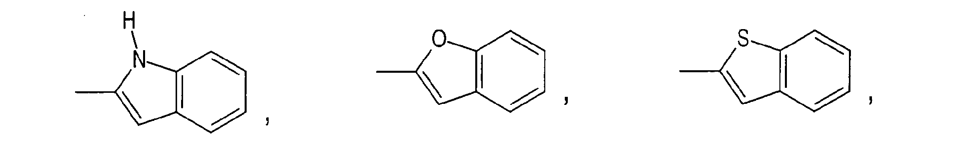 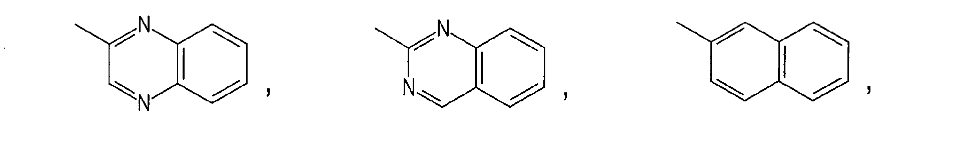 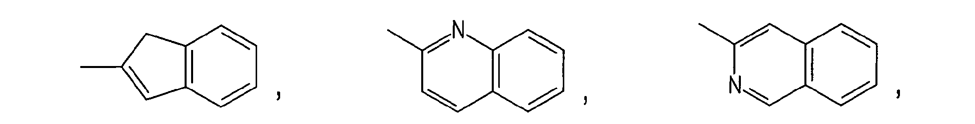 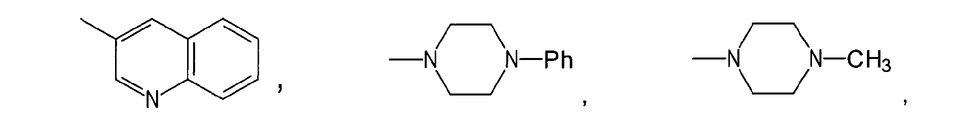 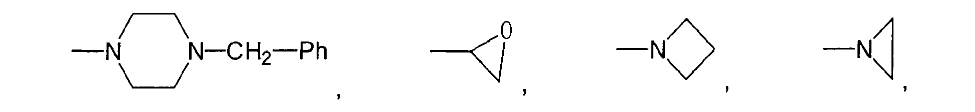 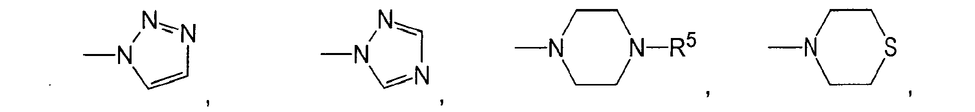 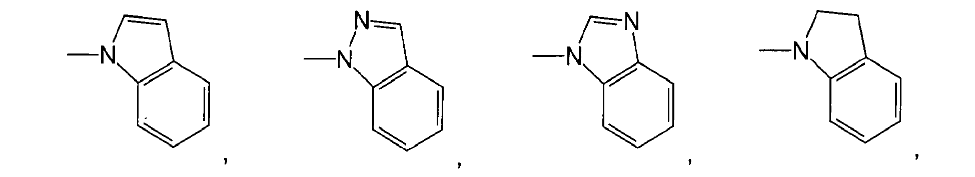 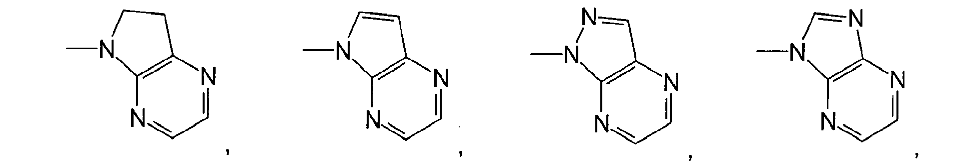 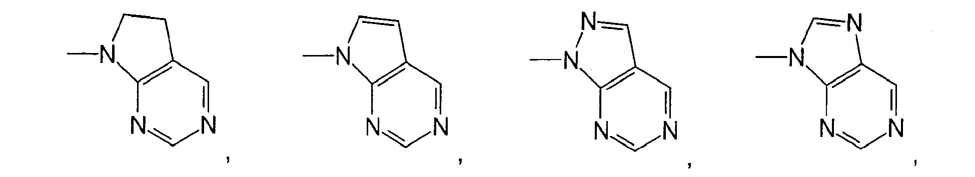 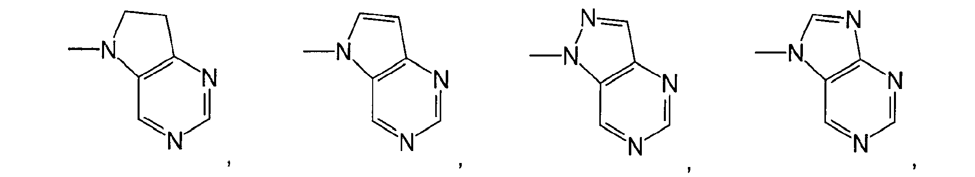 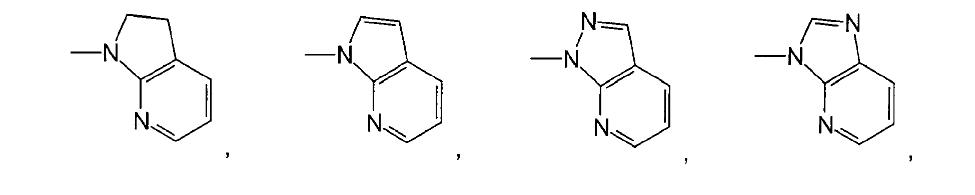 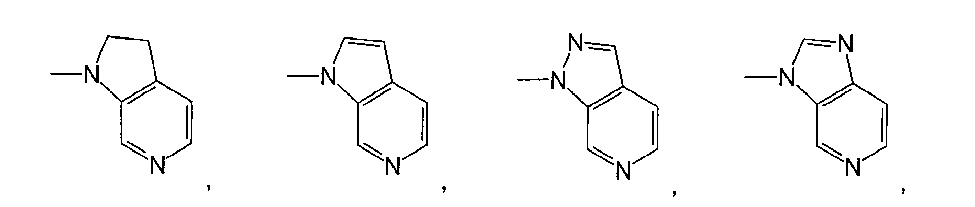 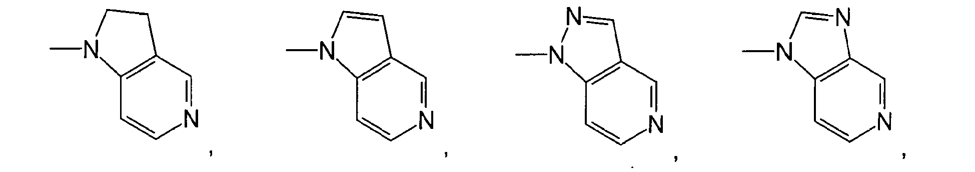 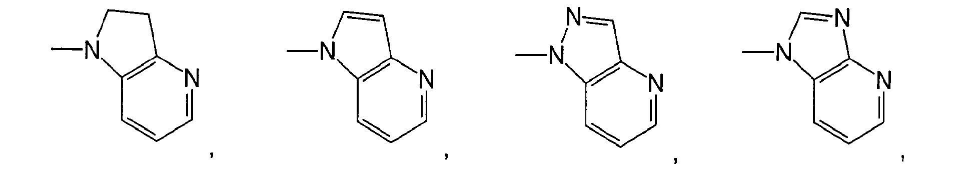 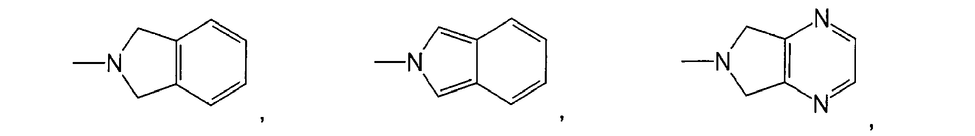 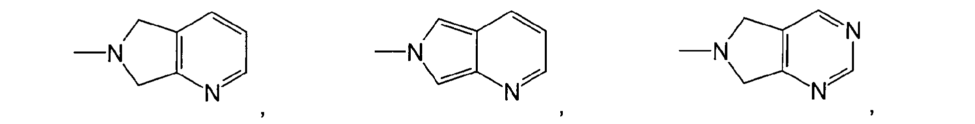 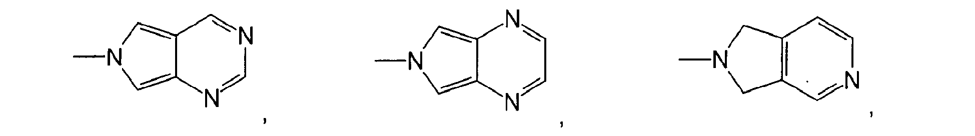 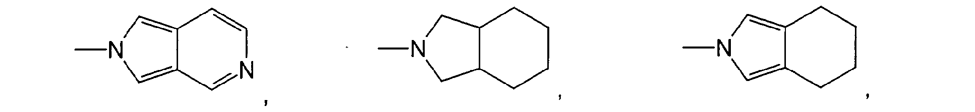 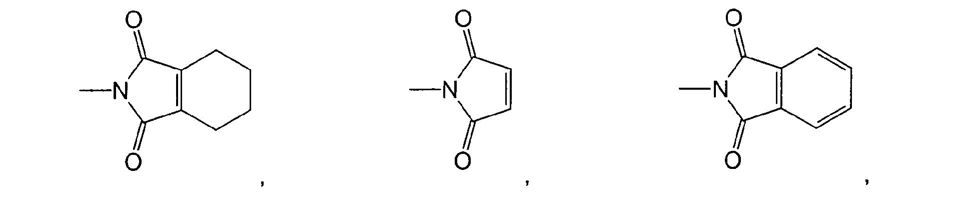
**R⁸- R¹⁶** represent independently of each other
   -H, -CH₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆CH=CH₂, -C₂H₄-CH=CH-CH₃, -CH₂CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, (CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, - CH(CH₃)-C₂H₄-CH=CH₂, -CH₂-CH(C=CH)₂, -C₂H₄-C(CH₃)=CH-CH₃, -CH₂CH(CH₃)-CH=CH-CH₃, -C≡C-C≡CH, -CH(CH₃)-CH₂CH=CH-CH₃, -CH₂-CH=CH-CH(CH₃)₂, -CH₂CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, - CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-C≡C-C≡CH, -CH(CH₃)-CH₂-C(CH₃)=CH₂, -CH(CH₃)-CH(CH₃)-CH=CH₂, -CH₂-C≡C-C≡CH, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂CH=CH-CH₃, -CH=CH-CH₂-CH=CH-CH₃, - CH(CH₃)-C(CH₃)=CH-CH₃, -C≡C-C≡C-CH₃, -CH=C(CH₃)-CH(CH₃)₂, -C(CH₃)=CH-CH(CH₃)₂, -CH(C≡CH)-C≡C-CH₃, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -CH(C≡CH)₂, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, - CH=CH-CH=CH-C₂H₅, -CH=CH-Ph, -CH₂-CH=CH-C(CH₃)=CH₂, -CH₂-CH=C(CH₃)-CH=CH₂, -C≡C-C≡C-C₂H₅, -CH₂-C(CH₃)=CH-CH=CH₂, -CH(CH₃)-CH₂-C≡CH, -CH₂-C≡C-CH₂-C≡CH, -CH(CH₃)-CH=CH-CH=CH₂, -CH=CH-CH₂-C(CH₃)=CH₂, -CH(CH₃)-C≡C-CH₃,-CH=CH-CH(CH₃)-CH=CH₂, -CH=C(CH₃)-CH₂-CH=CH₂, -C≡C-C₂H₄-C≡CH, -C₂H₄-CH(CH₃)-C≡CH, -C(CH₃)=CH-CH₂-CH=CH₂, -C≡C-CH(CH₃)-C≡CH, -CH=CH-CH=C(CH₃)₂, -CH₂-CH(CH₃)-CH₂-C≡CH, -C(C≡CH)₂-CH₃, -CH=CH-C(CH₃)=CH-CH₃, -CH=C(CH₃)-CH=CH-CH₃, -CH(C≡CH)-CH₂-C≡CH, -CH₂-CH(CH₃)-C≡CH, -C(CH₃)=CH-CH=CH-CH₃, -C₂H₄-C≡C-C≡CH, -CH=C(CH₃)-C(CH₃)=CH₂, -C(CH₃)=CH-C(CH₃)=CH₂, -C≡C-CH₂-C≡C-CH₃, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH(C₃H₇)-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C=C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅. -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C≡C-C≡C-CH₃, -CH₂-C(CH₃)₂-C≡CH, -CH(CH₃)-CH(CH₃)-C≡CH, -C(CH₃)(C₂H₅)-C≡CH, -Ph, -CH₂-Ph, 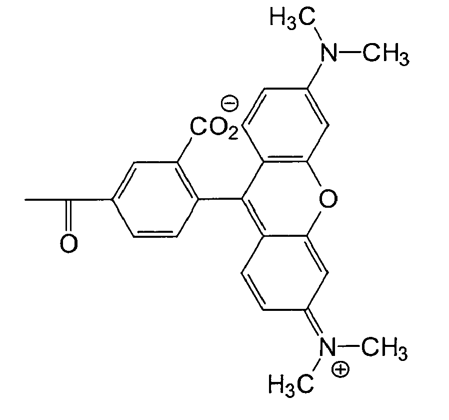
**k, I, m, n, p, r, s, t, u, v, w** and **x** represent independently of each other an integer 0, 1, 2, 3, 4, 5,6, 7, 8, 9, 10, 11, 12, 13, 14, 15 or 16;
and enantiomers, stereoisomeric forms, mixtures of enantiomers, diastereomers, mixtures of diastereomers, prodrugs, hydrates, solvates and racemates of the above mentioned compounds and pharmaceutically acceptable salts thereof.

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

Preferred are the following compounds of general formula (B): 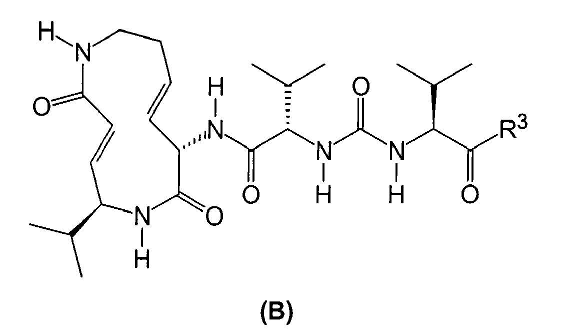 wherein
R³ represents -OH, -O-A-R⁴, -O-A-O-R⁸, -O-A-N(R⁸)(R¹⁰), -(CH₂CH₂O)ₘ-R⁸, -O-R⁸, -NH₂, -NH-A-R⁴, -NH-A-O-R⁸, -NH-A-N(R⁸)(R¹⁰), -NH-R⁸, -N(R⁸)(R¹⁰) or the residue -N(**R⁸**)(**R¹⁰**) represents a nitrogen heterocyclic ring selected from the residues as disclosed above and the substituents **R⁴, R⁸** and **R¹⁰** have the meanings as disclosed in above and m is an integer 0, 1, 2, 3, 4, 5,6, 7, 8, 9, 10, 11, 12, 13, 14, 15 or 16.

The preferred configuration of the iso-propyl group at the macrocyclic ring is S configuration and the preferred configuration of the two iso-propyl groups in neighbourhood of the urea residue is also S configuration. However, the S configuration especially for the two iso-propyl groups in neighbourhood of the urea residue is preferred but not essential and some or all of these iso-propyl groups could also have R configuration. The amide nitrogen attached to the macrocyclic ring has preferably S configuration.

Also preferred are compounds of general formula (C): 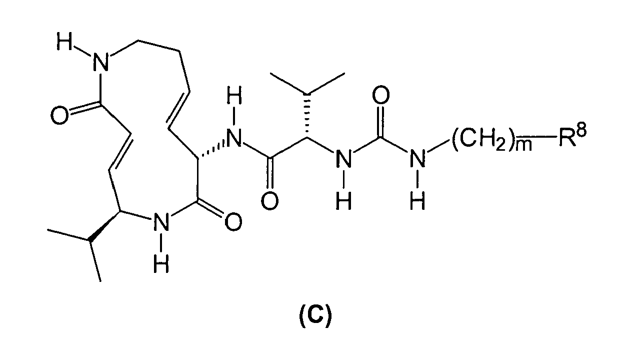 wherein **R⁸** and m have the meanings as disclosed above.

Still further preferred compounds are these of general formula (D): 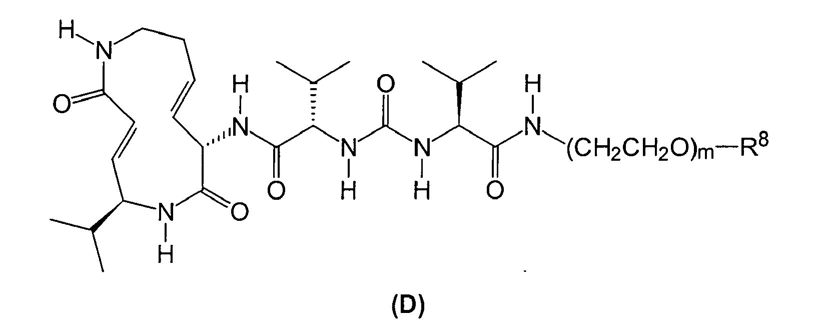 wherein **R⁸** and m have the meanings as disclosed above.

Further preferred compounds have general formula (E): 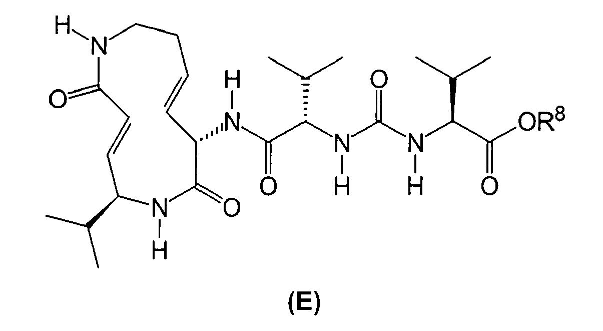 wherein **R⁸** has the meanings as disclosed above.

Most preferred are the compounds:
SyIA-OCH₃ (Compound 1),
SylA-Rhodamine (Compound 2),
SylA-PEG1 (Compound 3),
SylA-decanyl (Compound 4)
SylA-PEG2 (Compound 5)

Some of the compounds of any of the general formula (A) to (E) are basic and form pharmaceutically acceptable salts with organic and inorganic acids.

Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

Surprisingly, the inventive syringolin A derivatives exhibit highly potent properties as pharmaceutically active agents against diseases such as cancer or neurodegenerative diseases and disorders. Thus, the present invention relates to the compounds of any of the general formula (A) to (E) for use as pharmaceutically active agent in medicine.

Moreover it was figures out that the inventive compounds of any of the general formula (A) to (E) are potent tau aggregation inhibitors.

As mentioned above, the inventive syringolin A derivatives are highly potent pharmaceutically active compounds for treatment or prophylaxis of neurodegenerative diseases, neurodegenerative disorders, neurodegenerative conditions, proliferative diseases, tumors, cancer, polyarthritis, psoriasis, allograft rejection, transplant rejection, graft-versus-host disease, cardiovascular diseases and cardiovascular endothelial disorders.

Examples of neurodegenerative disease or neurodegenerative disorder or neurodegenerative condition are selected from the group comprising or consisting of Alzheimer Disease, Parkinson Disease, Huntington Disease, amyotrophic lateral sclerosis, AIDS-related dementia, retinitis pigmentosa, spinal muscular atrophy and cerebrellar degeneration, fragile X-associated tremor/ataxia syndrome (FXTAS), progressive supranuclear palsy (PSP), and striatonigral degeneration (SND), which is included with olivopontocerebellear degeneration (OPCD), and Shy Drager syndrome (SDS) in a syndrome known as multiple system atrophy (MSA).

One example of transplant rejection is the graft-versus-host-disease (GVHD) that can occur following bone marrow transplant. The donor's immune cells in the transplanted marrow make antibodies against the host's (transplant patient's) tissues and attack the patient's vital organs. Transplant rejections (also known as graft rejection or tissue/organ rejection) may commonly occur when tissue or organs which need blood supply are transplanted. Said organs comprise especially inner organs such as heart, heart-lungs, lungs, liver, kidney, pancreas, spleen, skin, tissue, bone marrow, spinal marrow, hormone producing glands, gonads and gonadal glands.

The inventive compounds showed an activation of a protective defence response in cardiovascular endothelial disorders, which makes these compound suitable for the treatment and prophylaxis of cardiovascular diseases.

Examples of cardiovascular diseases and or cardiovascular endothelial disorders are: adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, atherosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

Examples of proliferative disease or tumor or cancer are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

The following Table I shows the Kᵢ' values of some selected inventive compounds in regard to chymotryptic activity, tryptic activity and caspase-like activity.

**Table I: Inhibitory effect of the inventive compounds (+++ = Kᵢ' value ≤ 20 nM; ++ = 20 nM < Kᵢ' value < 100 nM; + = Kᵢ' value ≥ 100 nM; - = Kᵢ' value ≥ 50 µM)**

| ① | ② | ③ | ④ |
|---|---|---|---|
| 1 | **+** | **+** | **-** |
| 2 | **+** | **+** | **-** |
| 3 | **+++** | **++** | **+** |
| 4 | **+++** | **++** | **+** |
| 5 | **+++** | **++** | **+** |

| | | | |
|---|---|---|---|
| ① compound number ② chymotryptic activity [Kᵢ' value in nM] ③ tryptic activity [Kᵢ' value in nM] ④ caspase-like activity [Kᵢ' value in nM] | | | |

The Kᵢ' value is defined as the apparent dissociation constant of the inhibitor.
The Kᵢ' value can be calculated from the IC₅₀ value using the equation: Kᵢ' = IC₅₀ * Km / (S+Km), wherein S is the concentration of the substrate, and Km is the substrate concentration (in the absence of an inhibitor) at which the velocity of the reaction is half-maximal. The Kᵢ' of an inhibitor for inhibition of a particular substrate (fixed Km) is constant.

As used herein, the term "chymotryptic activity" refers to inhibition of the human 20S proteasome measured by the reduced proteolytic cleavage of the "chymotryptic" proteasome substrate Suc-LLVY-AMC.

As used herein, the term "tryptic activity" refers to inhibition of the human 20S proteasome measured by the reduced proteolytic cleavage of the "tryptic" proteasome substrate Boc-LRR-AMC.

As used herein, the term "caspase-like activity" refers to inhibition of the human 20S proteasome measured by the reduced proteolytic cleavage of the "caspase-lie" proteasome substrate Z-LLE-AMC.

### Pharmaceutical compositions

Another aspect of the present invention relates to pharmaceutical formulations and pharmaceutical compositions containing at least one syringolin A derivative as an active ingredient, together with at least one pharmaceutically acceptable carrier, excipient, solvent and/or diluents.

The compounds of any of the general formula (A) to (E) can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. The preferred preparations and formulations are in administratable form which is suitable for oral application. These administratable forms, for example, include pills, tablets, film tablets, coated tablets, capsules, powders and deposits. Other than oral administratable forms are also possible. The inventive syringolin A derivatives or pharmaceutical preparations or formulations containing said syringolin A derivatives may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

Within the disclosed methods the pharmaceutical compositions of the present invention, containing at least one syringolin A derivative of any of the general formula (A) to (E) or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive syringolin A derivatives of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophilic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the syringolin A derivatives compounds of the present invention may be found in "Remington's Pharmaceutical Sciences" Mack Publishing Co., Easton PA. A suitable composition comprising at least one compound of the invention and/or pharmaceutically acceptable salts thereof may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of any of the general formula (A) to (E) refers to that amount of the compound that results in an at least partial inhibition of a target molecule. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. More preferably, the dosage of the compound corresponds to an effective concentration in the range of 0.1 nM - 10 µM. The actual amount of the composition administered will be dependent on the subject being treated, on the subject's weight, the severity of the affliction, the manner of administration and the judgement of the prescribing physician.

Preferred is the administration of at least one compound of any of the general formula (A) to (E) together with at least one drug selected from the group comprising or consisting of meclofenoxate, nicergoline, piracetame, pyritinole, tacrine, donezepiel, galantamine, rivastigmine, memantine, IMPase-inhibitors and extracts from Gingko biloba. Consequently, pharmaceutical formulations and pharmaceutical compositions are preferred which contain at least one compound of any of the general formula (A) to (E) together with at least one drug selected from the group comprising or consisting of meclofenoxate, nicergoline, piracetame, pyritinole, tacrine, donezepiel, galantamine, rivastigmine, memantine, IMPase-inhibitors and extracts from Gingko biloba.

### Chemical synthesis of syringolin A derivatives

The present invention relates to the full chemical synthesis of syringolin A derivatives according to the synthetic route disclosed in detail in the following.

The chemical synthesis starts from the protected amino acid valine. Common amino protecting groups such as Boc (tert-butoxycarbonyl), Fmoc (9-fluorenylmethoxycarbonyl) or Troc (N-trichloroethoxycarbonyl) protecting groups can be used while the Troc group is preferred and an ester protecting group such as a methyl ester is preferred for the carboxylic acid group.

Thus the chemical synthesis starts with compound (IIIA) wherein PG and PG' refer independently of each other to a suitable protecting group.

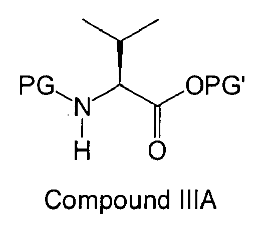

Compound IIIA is reacted at low temperatures preferably below -50°C and more preferably below -70°C with a strong base such as DIBAL-H and thereafter with Ph₃P=COOPG" in a suitable solvent such as methylenchloride (DCM), tetrahydrofurane (THF), chloroform or the like in order to obtain compound IIIB. In Ph₃P=COOPG" the group PG" refers to a suitable protecting group such as methyl (Me). PG refers preferably to a Troc protecting group.

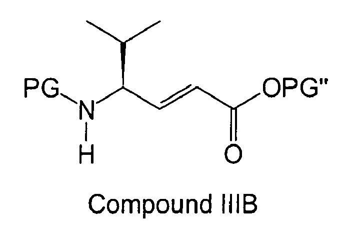

Compound IIIB is reacted with osmium tetroxide (OsO₄), sodium periodate (NaIO₄) and N-methylmorpholine-N-oxide (NMO) in acetone / water (2:1) and a ketal ring is subsequently formed using 2,2-dimethoxypropane (2,2-DMP) and pyridinium p-toluene sulfonate (PPTS) in DCM under reflux in order to obtain compound IIIC.

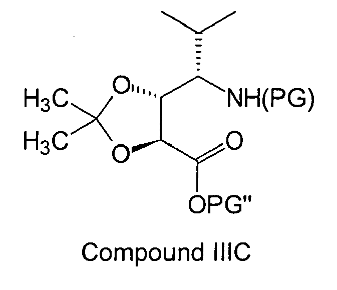

Compound IIIC is deprotected under basic conditions using for example lithium hydroxide (LiOH) in methanol / water mixture and is subsequently reacted with 1-aminobut-3-en hydrochloride preferably in presence of PyBOP (benzotriazol-1-yl-oxytripyrrolidinophosphonium hexafluorophosphate), DIEA (N,N-diisopropylethylamine) and 1-hydoxybenzotriazole (HOBt) in a polar aprotic solvent such as methylene chloride (DCM) in order to obtain compound IIID.

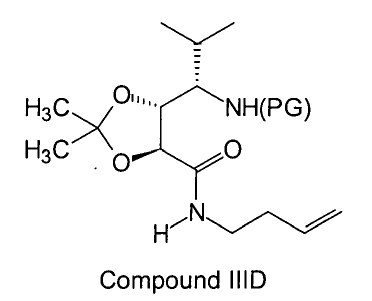

Compound IIID is then deprotected for instance by means of 2,6-lutidine and trimethylsilyl trifluoromethanesulfonate (TMSOTf) in a polar aprotic solvent such as DCM, THF, chloroform to synthesize compound III and thereafter compound III was reacted with acid II 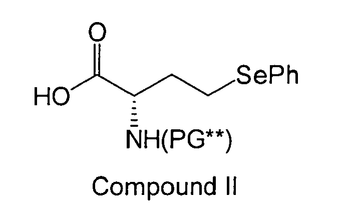 which is obtained from the corresponding protected homoserine lactone (I) by sodium borohydride reduction. The reaction between compound II and III was performed under addition of PyBOP, DIEA and 1-hydroxy-7-azabenzotriazole (HOAt) in a polar aprotic solvent such as DCM in order to obtain compound IV wherein PG** refers to a suitable amino protecting group such as Troc

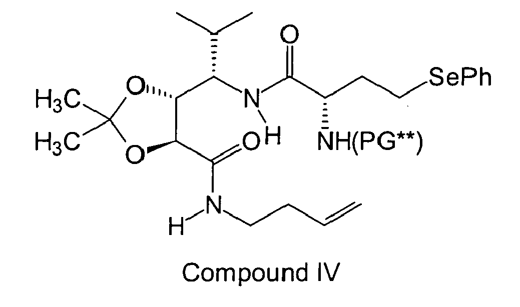

Thereafter an oxidation step with hydrogen peroxide is performed preferably in the presence of DIEA and in a polar aprotic solvent such as THF or DCM in order to produce compound V.

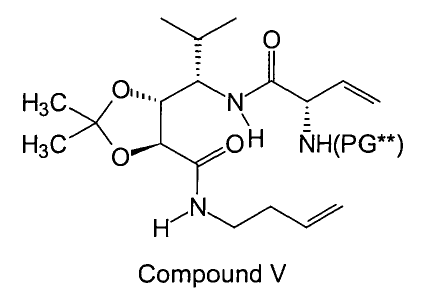

Now as key step a macrolactamisation is carried out using the Grubbs II catalyst (Sigma-Aldrich, catalog No. 569747-2g) preferably in toluene and at elevated temperatures preferably between 80°C and 100°C to obtain compound VI

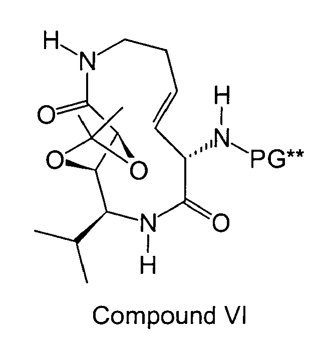

Now the ketal group of compound VI is cleaved with an organic acid such as formic acid in water / THF or with p-TsOH in a methanol water mixture under microwave treatment (120W) for 1 to 2 hours and thereafter the vicinal dihydroxy group is converted to a thiocarbonate group be use of CS(Im)₂ and 4-(dimethylamino)-pyridine (DMAP) in a polar aprotic solvent such as THF to obtain compound VII.

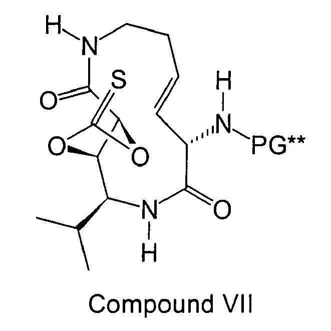

The thiocarbonate group of compound VII was reduced with a phosphite to the double bond of compound VIII.

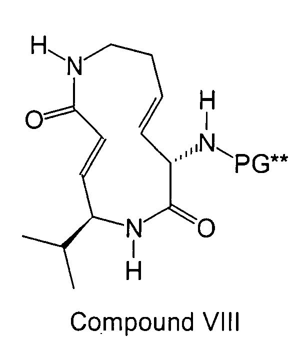

Now compound VIII is deprotected by suitable reaction conditions which are appropriate for the used protecting group and which do not destroy the macrocyclic ring system. In the case of the preferred Troc protecting group zinc in THF and acetic acid was used in order to obtain compound IX.

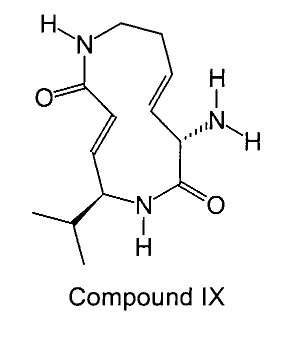

Compound IX is reacted with the amino protected amino acid valin under common conditions for forming an amide bond such as PyBOP, DIEA, 1-hydroxy-7-azabenzotriazole (HOAt) in DMF in order to obtain compound X.

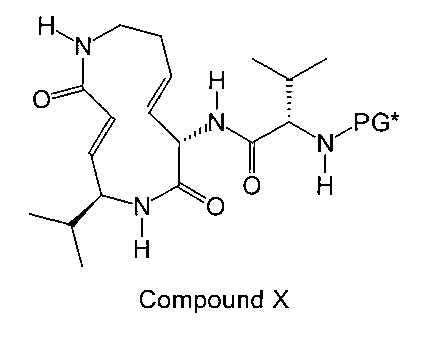

PG* refer to a suitable amino protecting group such as BOC. After formation of the amide bond, the amino protecting group is cleaved under suitable conditions which do not destroy the macrocyclic ring system. In the case of a BOC protecting group trifluoroacetic acid in dichloromethane is used and the deprotected compound XI is obtained in almost quantitative yield.

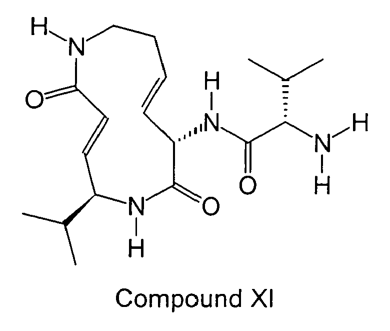

This key compound XI having a free amino group was than used to synthesize additional derivatives, such as for example the decyl urea compound 4 (SylA-decanyl) by a two-step process.

### Description of Figures

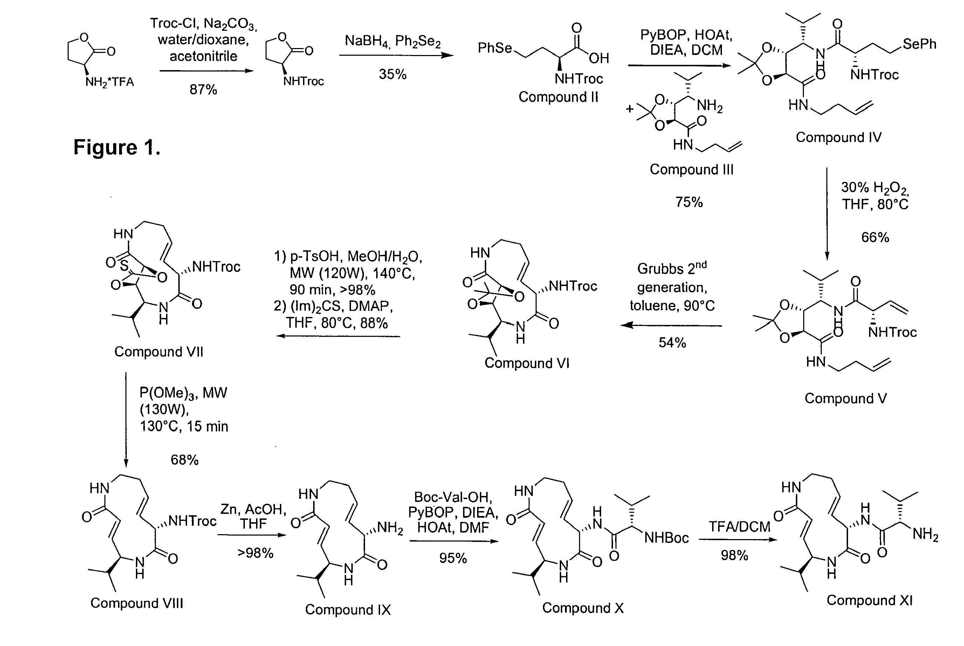

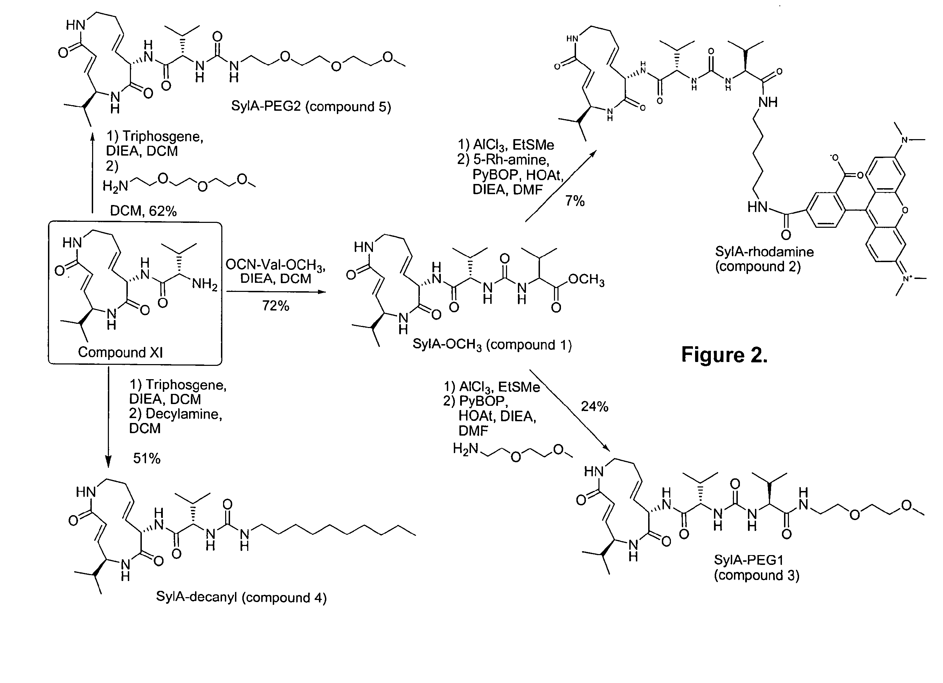

- Fig. 1: Shows a synthetic route for synthesizing the key compound XI which is the SylA macrocycle attached to a valine residue. Starting from this intermediate, several derivatives can be prepared as shown in Fig. 2.
- Fig. 2: shows a variety of synthetic pathways for synthesizing the corresponding syringolin A derivatives compound 1-5.

### Examples

### Example 1: Synthesis of key intermediate XI

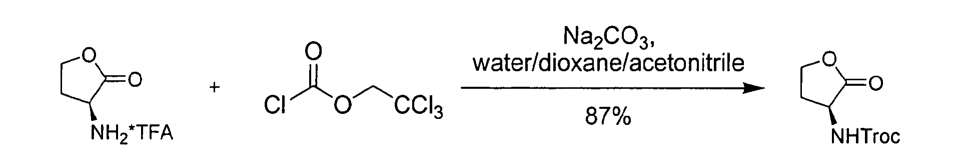

Homoserine lactone salt (1.5 g, 14.9 mmol, 1 eq.) and sodium carbonate (1.6 g, 14.9, 1 eq.) were dissolved in a mixture water/dioxane/acetonitrile (240 mL/180 mL/150 mL) and the solution was cooled to 0°C. A solution of 2,2,2-trichloroethyl chloroformate (2.2 mL, 16.4 mmol, 1.1 eq.) in dioxane (200 mL) was then added *via* an addition funnel over 30 min. The mixture was stirred for 24h at room temperature. The solvents were removed *in vacuo* and the remaining residue was partitioned between a saturated ammonium chloride solution (200 mL) and dichloromethane (200 mL). The aqueous layer was extracted 3 times more with dichloromethane and the combined organic layers were finally dried over Na₂SO₄. After concentration the crude product was purified by flash column chromatography (40% ethyl acetate in cyclohexane) to afford 3.58 g (12.95 mmol, 87%) of compound (I) as a colorless solid.

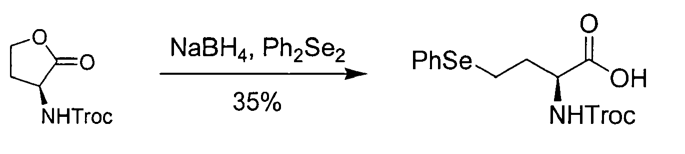

Sodium borohydride (446 mg, 11.8 mmol, 4.4 eq.) was disposed under argon in a 100 mL flame dried flask. A solution of diphenyl diselenide (3.34 g, 10.7 mmol, 2 eq.) in dimethylformamide (100 mL) was added via cannula, followed by addition of a solution of Troc-homoserine lactone (I) (2.96 g, 10.7 mmol, 1 eq.) in dimethylformamide (100 mL). The resulting mixture was heated to 100 °C for 2 hours. After cooling to 0 °C, methanol (20 mL) was added and the mixture was stirred for an hour. The solvents were removed in vacuo and the remaining residue was partitioned between ethyl acetate (250 mL) and 100 mM NaOAc buffer (pH 5.0). The aqueous layer was extracted twice more with ethyl acetate (200 mL). The combined organic layers were dried over Na₂SO₄, filtered and concentrated. The crude product was purified by flash column chromatography (40% ethyl acetate in cyclohexane) to afford 1.60 mg (3.69 mmol, 35%) of (II) as a colorless oil.

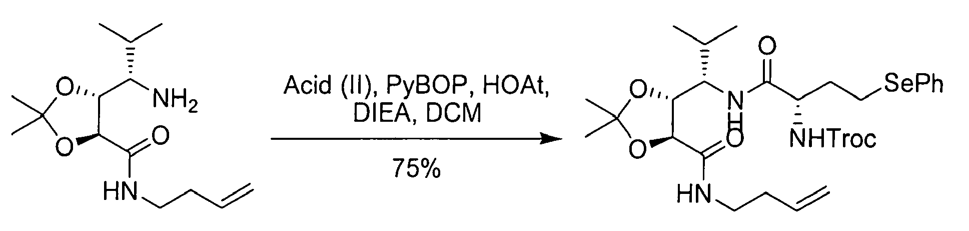

Acid (II) (1.5 g, 3.46 mmol, 1 eq.), amine (III) (936 mg, 3.46 mmol, 1 eq.), PyBOP (2.70 g, 5.19 mmol, 1.5 eq.) and HOAt (706 mg, 5.19 mmol, 1.5 eq.) were dissolved in dichloromethane (15 mL) in a 25 mL flask. The solution was cooled to 0°C and N,N-diisopropylethylamine (1.2 mL, 6.92 mmol, 2 eq.) was added. The reaction was stirred overnight at room temperature, quenched by addition of a 20% aq. citric acid solution and extracted with chloroform (3×50 mL). The combined organic layers were dried over Na₂SO₄, filtered and concentrated to dryness. The crude product was purified by flash column chromatography (50% diethyl ether in petroleum ether) to afford 1.77 g (2.59 mmol, 75%) of (IV) as a colorless solid.

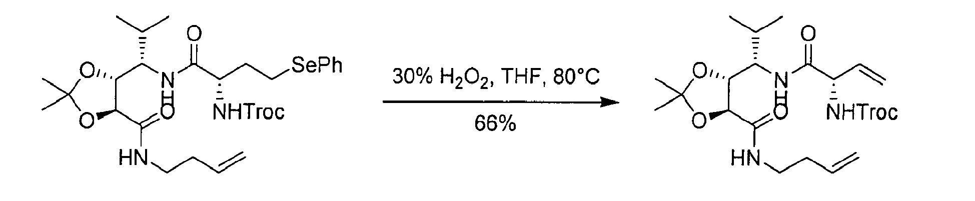

(IV) (1.7 g, 2.48 mmol) was dissolved in tetrahydrofurane (145 mL) in a 250 mL flask. Hydrogen peroxide (30% in water, 2.8 mL) was added and the resulting mixture was heated to 80 °C for 90 min. The reaction was diluted with ethyl acetate and washed with brine twice (2×50 mL). The organic layer was then dried over Na₂SO₄, filtered and concentrated to dryness. The crude product was purified by flash column chromatography (50% diethyl ether in petroleum ether) to afford 861 mg (1.63 mmol, 66%) of (V) as a colorless solid.

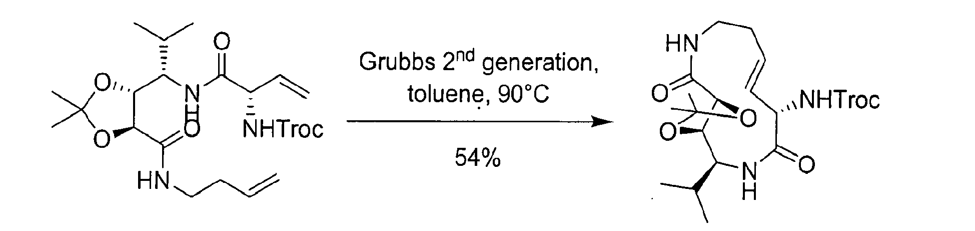

(V) (794 mg, 1.500 mmol, 1 eq.) was dissolved under argon in toluene (750 mL) in a 1 L flame-dried flask and heated to 90°C. A solution of Grubbs' 2^{nd} generation catalyst (192 mg, 0.225 mmol, 0.15 eq.) in toluene (25 mL) was added over 1 hour with a syringe pump to the preheated mixture. The resulting solution was stirred for further 2 hours at 90°C. After concentration to dryness, the crude product was purified by flash column chromatography (50% ethyl acetate in cyclohexane) to afford 405 mg (0.809 mmol, 54%) of (VI) as a light brown solid.

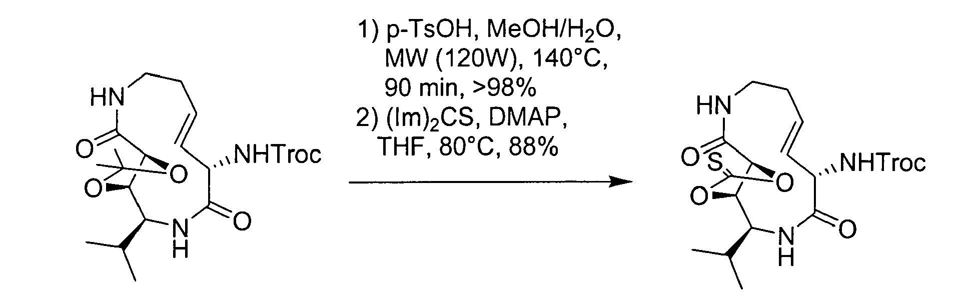

In a 10 mL vessel was placed (VI) (20 mg, 40 µmol, 1 eq.), *p*-toluenesulfonic acid monohydrate (7.6 mg, 40 µmol, 1 eq.), methanol/water (3:1, 5 mL) and a magnetic stirring bar. The vessel was sealed with a septum, placed into the MW cavity, and locked with the pressure device. Constant microwave irradiation of 120 W as well as a simultaneous air-cooling (300 kPa, 45 Psi) were used during the entire reaction time (90 min, 140 °C, resulting reaction pressure 12 bar). After cooling to room temperature, the solvent was removed under reduced pressure to afford 18 mg (39 µmol, >98%) of the dihydroxyl intermediate as a colorless solid. The product was pure enough to be used in the next step without further purification.
After performing this reaction several times, all product fractions were pooled and the resulting residue of the dihydroxyl derivative (124 mg, 269 µmol, 1 eq.) was dissolved under argon in tetrahydrofurane (80 mL) in a 250 mL flame-dried flask. To this solution was added thiocarbonyl diimidazole (480 mg, 2.69 mmol, 10 eq.) and 4-(dimethylamino)pyridine (329 mg, 2.69 mmol, 10 eq.). The resulting reaction mixture was heated to 80°C and stirred at this temperature overnight. After cooling to room temperature, a small portion of silica gel was added and the solvent was removed under reduced pressure. The adsorbed crude product was purified by flash column chromatography (50% ethyl acetate in cyclohexane) to yield 119 mg (237 µmol, 88%) of (VII) as a colorless solid.

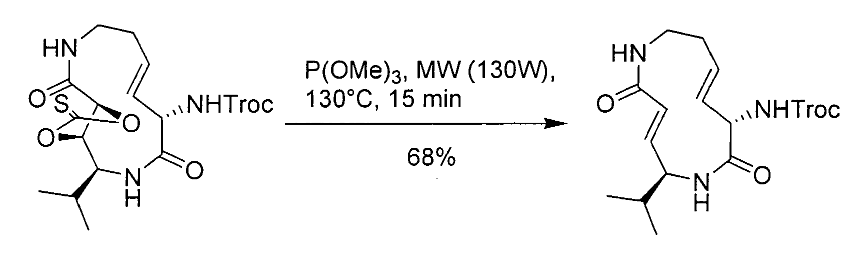

In a 10 mL vessel was placed (VII) (60 mg, 119 µmol, 1 eq.), trimethyl phosphite (1.2 mL) and a magnetic stirring bar. The vessel was sealed with a septum, placed into the MW cavity, and locked with the pressure device. Constant microwave irradiation of 130 W as well as a simultaneous air-cooling (300 kPa, 45 Psi) were used during the entire reaction time (15 min, 130 °C, resulting reaction pressure 2 bar). After concentration to dryness, the crude product was purified by flash column chromatography (6% methanol in dichloromethane) to yield 34 mg (81 µmol, 68%) of (VIII) as a colorless solid.

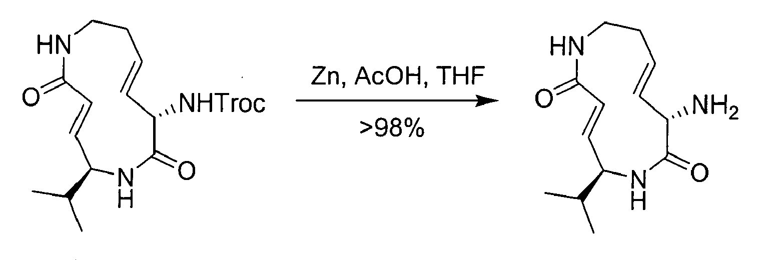

(VIII) (34 mg, 81 µmol, 1 eq.) was dissolved in tetrahydrofurane (1 mL) in a 10 mL flask. Acetic acid was added (1 mL), followed by zinc powder (798 mg, 12.2 mmol, 150 eq.) which was added in portions over 30 minutes. After 3 hours of vigorous stirring, the mixture was filtered over a small plug of Celite and washed with ethyl acetate. After evaporation to dryness, 20 mg (79 µmol, 98%) of (IX) was obtained as a colorless solid.

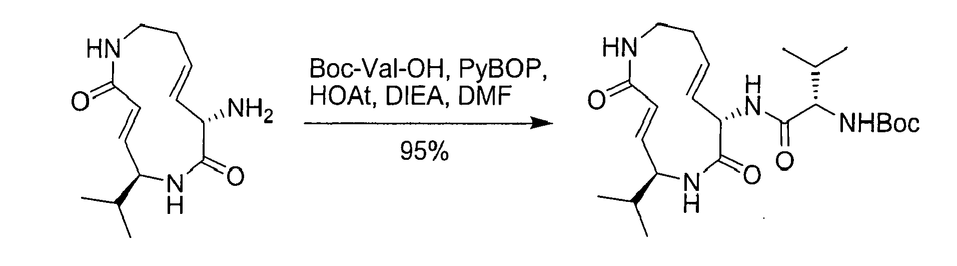

Boc-Valine-OH (21 mg, 95 µmol, 1.2 eq.), amine (IX) (20 mg, 79 µmol, 1 eq.), PyBOP (62 mg, 119 µmol, 1.5 eq.) and HOAt (16 mg, 119 µmol, 1.5 eq.) were dissolved in N,N-dimethylformamide (1.5 mL) in a 10 mL flask. The solution was cooled to 0°C and N,N-diisopropylethylamine (28 µL, 158 µmol, 2 eq.) was added. The reaction was stirred for 1 hour at room temperature. After concentration to dryness, the crude product was purified by flash column chromatography (6% methanol in dichloromethane) to yield 34 mg (75 µmol, 95%) of (X) as a colorless solid.

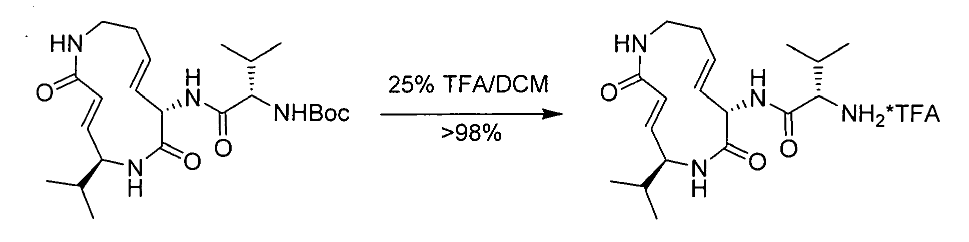

(X) (34 mg, 75 µmol, 1 eq.) was dissolved in a mixture trifluoroacetic acid/dichloromethane (1:3, 1 mL) in a 10 mL flask. After 30 minutes, the mixture was evaporated to dryness to afford 34 mg (73 µmol, 98%) of (XI) as a colorless solid.

### Example 2: Synthesis of SylA-OCH₃ (compound 1)

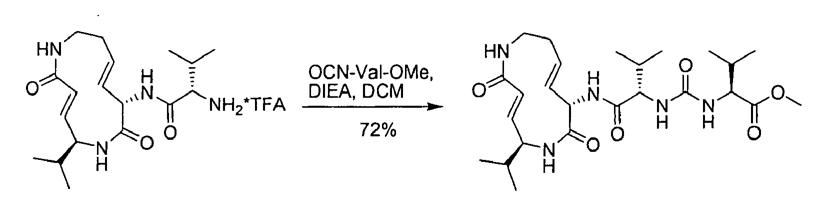

(XI) (29 mg, 63 µmol, 1 eq.) was dissolved in dichloromethane (2 mL) and *N,N-*diisopropylethylamine (22 µL, 126 µmol, 2 eq.). Then, a solution of methyl (S)-(-)-2-isocyanato-3-methylbutyrate (18 µL, 126 µmol, 2 eq.) in dichloromethane (500 µL) was added in one portion. The resulting mixture was stirred overnight and concentrated to be purified by preparative HPLC to yield 23 mg (45 µmol, 72%) of compound 1 (SylA-OCH₃) as a colorless solid.

### Example 3: Synthesis of SylA-Rhodamine (compound 2)

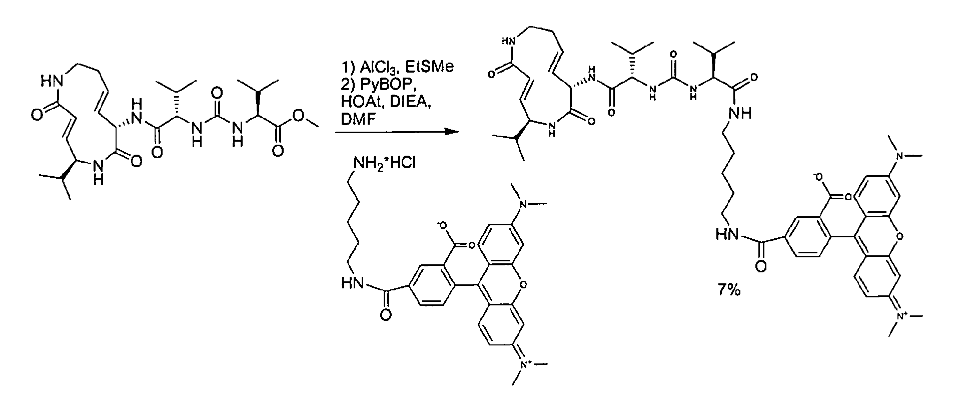

Compound 1 (SylA-OCH₃, 10 mg, 20 µmol, 1 eq.) was dissolved in ethylmethyl sulfite (500 µL) and trichloro aluminium (13 mg, 100 µmol, 5 eq.) was added in one portion. The mixture was stirred for 10 h and quenched with some drops of water. After concentration, a solution of rhodamine-pentylamine HCl salt (5-isomer) (22 mg, 40 µmol, 2 eq.), PyBOP (26 mg, 50 µmol, 2.5 eq.), HOAt (7 mg, 50 µmol, 2.5 eq.) and DIEA (17 µL, 100 µmol, 5 eq.) in DMF (500 µL) was added. The resulting mixture was stirred overnight and concentrated to be purified by preparative HPLC to yield 1.4 mg (1.4 µmol, 7%) of compound 2 (SylA-Rhodamine) as a purple solid.

### Example 4: Synthesis of SylA-PEG1 (compound 3)

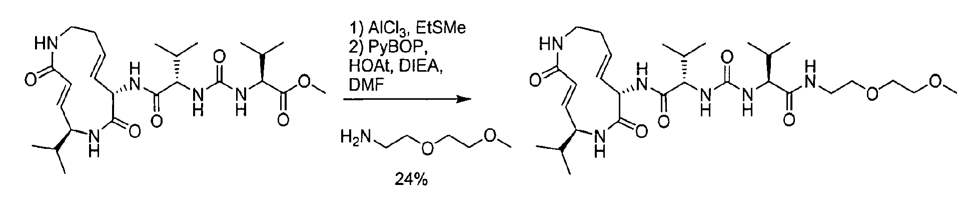

Compound 1 (SylA-OCH₃, 10 mg, 20 µmol, 1 eq.) was dissolved in ethylmethyl sulfite (500 µL) and trichloro aluminium (13 mg, 100 µmol, 5 eq.) was added in one portion. The mixture was stirred for 10 h and quenched with some drops of water. After concentration, a solution of 2-(2-methoxyethoxy)ethanamine (4.8 mg, 40 µmol, 2 eq.), PyBOP (26 mg, 50 µmol, 2.5 eq.), HOAt (7 mg, 50 µmol, 2.5 eq.) and DIEA (14 µL, 80 µmol, 4 eq.) in DMF (500 µL) was added. The resulting mixture was stirred overnight and concentrated to be purified by preparative HPLC to yield 2.8 mg (4.7 µmol, 24%) of compound 3 (SylA-PEG1) as a colorless solid.

### Example 5: Synthesis of SylA-decanyl (compound 4)

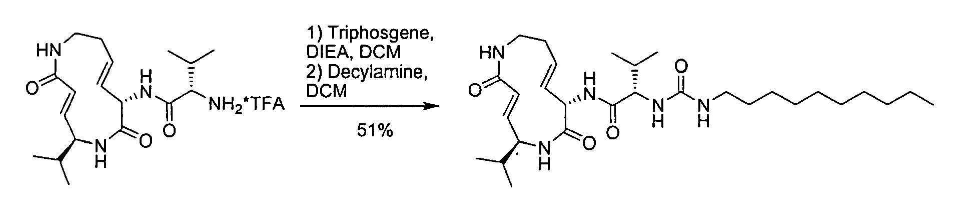

Triphosgene (65 mg, 219 µmol, 3 eq.) was dissolved in dichloromethane (500 µL). To this solution was added dropwise a solution of (XI) (34 mg, 73 µmol, 1 eq.) in dichloromethane (2 mL) and N,N-diisopropylethylamine (25 µL, 146 µmol, 2 eq.). The reaction was stirred for 30 minutes at room temperature. Then, a solution of decylamine (145 µL, 730 µmol, 10 eq.) in dichloromethane (500 µL) was added in one portion. The resulting mixture was stirred overnight and concentrated to be purified by preparative HPLC to yield 20 mg (37 µmol, 51%) of compound 4 (SylA-decanyl) as a colorless solid.

### Example 6: Synthesis of SylA-PEG2 (compound 5)

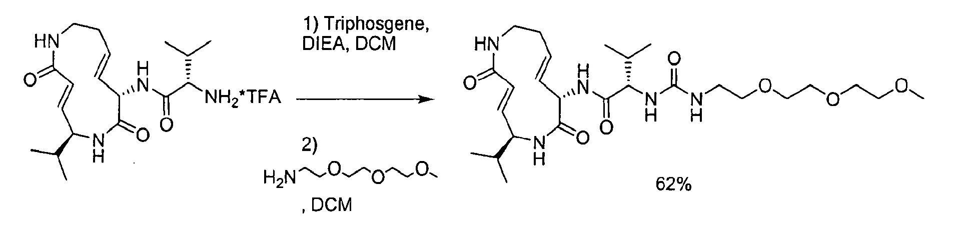

Triphosgene (65 mg, 219 µmol, 3 eq.) was dissolved in dichloromethane (500 µL). To this solution was added dropwise a solution of (XI) (34 mg, 73 µmol, 1 eq.) in dichloromethane (2 mL) and N,N-diisopropylethylamine (25 µL, 146 µmol, 2 eq.). The reaction was stirred for 30 minutes at room temperature. Then, a solution of H₂N-(CH₂)₂-(OCH₂CH₂)₂-OCH₃ (119 mg, 730 µmol, 10 eq.) in dichloromethane (500 µL) was added in one portion. The resulting mixture was stirred overnight and concentrated to be purified by preparative HPLC to yield 24 mg (45 µmol, 62%) of compound 5 (SylA-PEG2l) as a colorless solid.

### Example 7: Measurement of the chymotryptic activity

Proteasome inhibition assays were performed with human erythrocyte 20S proteasomes (AK-740, Biomol) at 37 °C in 100 µL reaction volumes containing 2 µg ml⁻¹ 20 proteasome and 100 µM Suc-LLVY-AMC as a substrate. Cleavage efficience in presence of varying inhibitor concentrations were followed by an MWGt Sirius HT plate reader (BIO-TEK instruments) equipped with 360 nm excitation and 460 nm emission filters. The obtained fluorescence values (RFU) were plotted versus time (t). The fluorescence data were fitted by the least squares method using SigmaPlot 10.0 software (Systat Software) to the equation f = f₀ + Vₛₜ + [(vᵢ - vₛ)/k_{obs}][1-exp(-k_{obs}*t)], where vᵢ and vₛ are initial and final velocities, respectively, and k_{obs} is the pseudo-first order associaten rate constant. Kᵢ' values were then determined by plotting vᵢ against inhibitor concentration [I]. Using SigmaPlot, the data were fitted with the hyperbolic equation vᵢ = c + [v₀/(Kᵢ' + [I])] where v₀ is the velocity of the control.

### Example 8: Measurement of the tryptic activity

The inhibition of the tryptic proteasome activity was performed in analogy to example 7 with using Boc-LRR-AMC as a substrate instead of Suc-LLVY-AMC.

### Example 9: Measurement of the caspase-like activity

The inhibition of the caspase-like proteasome activity was performed in analogy to example 7 with using Z-LLE-AMC as a substrate instead of Suc-LLVY-AMC.

## Claims

1. Compound having the general formula (A): 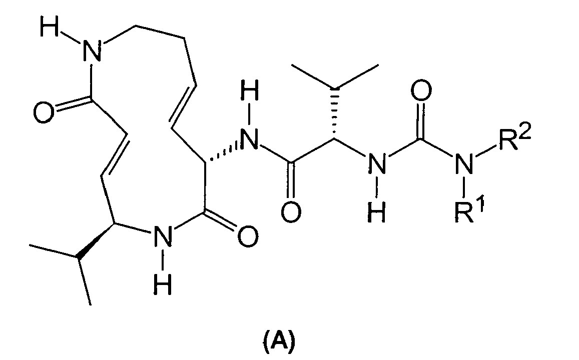 wherein
**R¹** and **R²** represent independently of each other:
-H, -(CH₂)ₖ-C(R¹²)(R¹³)-(CH₂)ₗ-C(R¹⁴)(R¹⁵)(R¹⁶), -A-R⁴, -A*-R⁵, -A-R⁸, -A*-R⁹, -A-O-R⁸, -A*-O-R⁹, -A-N(R⁸)(R¹⁰), -A*-N(R⁹)(R¹¹), 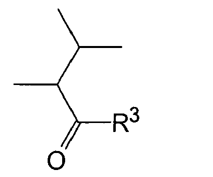 -(CH₂CH₂O)ₘ-R⁸, -(CH₂CH₂O)ₙ-R⁹, -R⁸, -R⁹;
**R³** represents -OH, -O-A-R⁴, -O-A-O-R⁸, -O-A-N(R⁸)(R¹⁰), -(CH₂CH₂O)ₘ-R⁸, -O-R⁸, -NH₂, -NH-A-R⁴, -NH-A-O-R⁸, -NH-A-N(R⁸)(R¹⁰), -NH-R⁸, -N(R⁸)(R¹⁰);
or the residues -N(**R¹**)(**R²**), -N(**R⁸**)(**R¹⁰**), -N(**R⁹**)(**R¹¹**) represent independently of each other a nitrogen heterocyclic ring selected from: 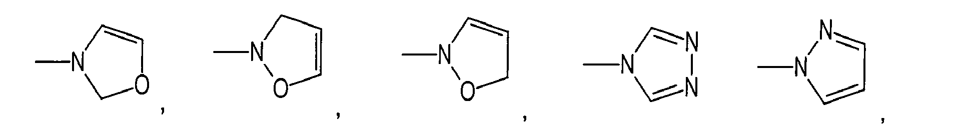 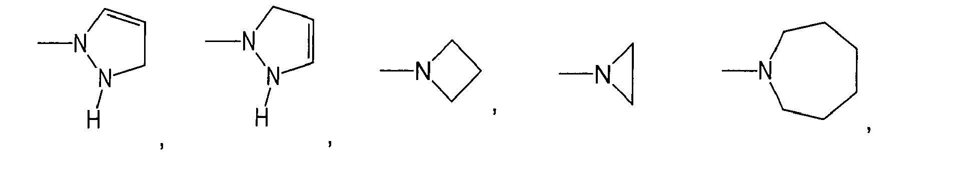 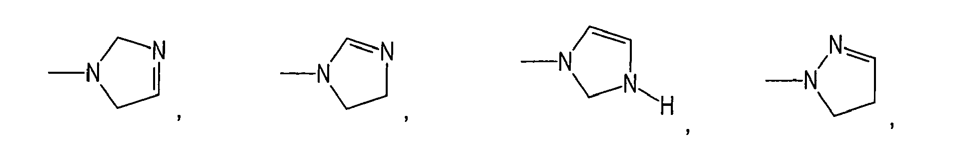 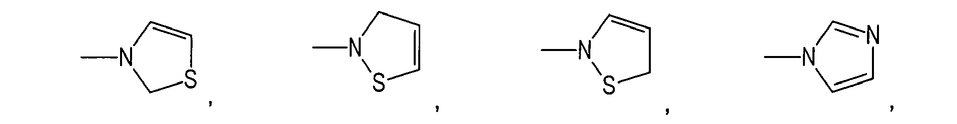 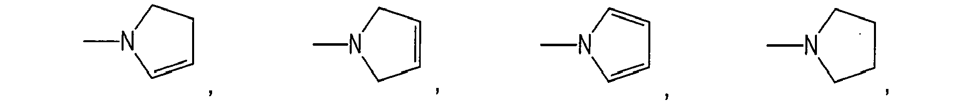 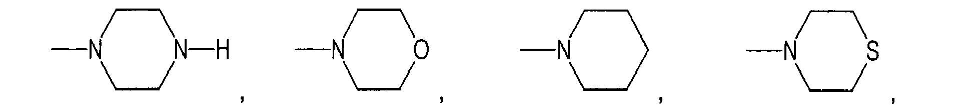 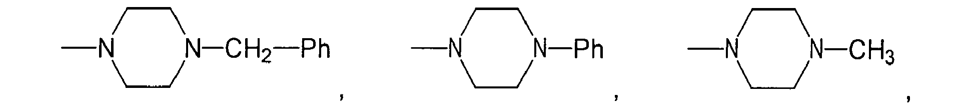 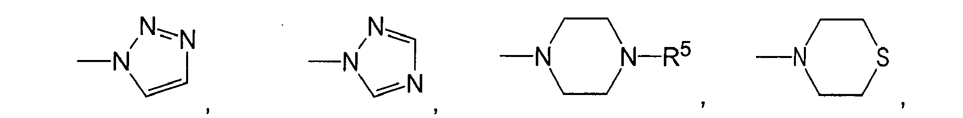 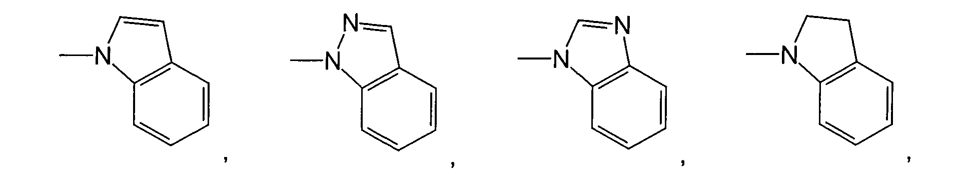 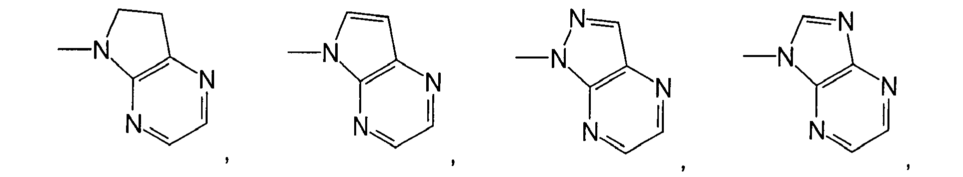 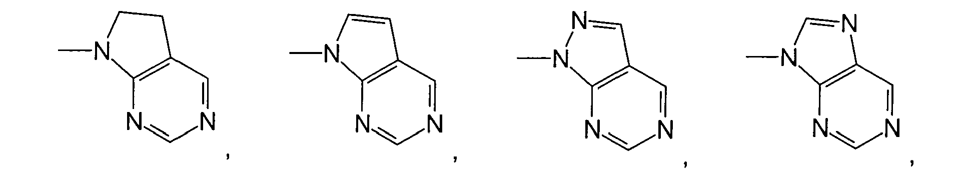 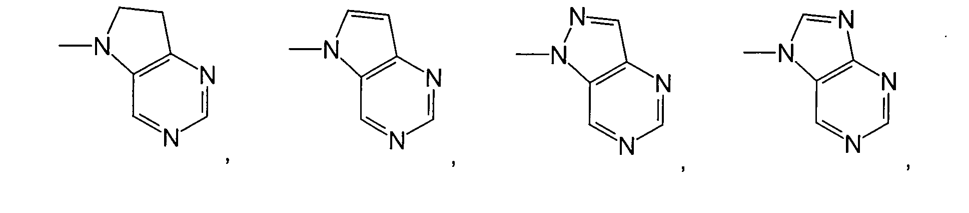 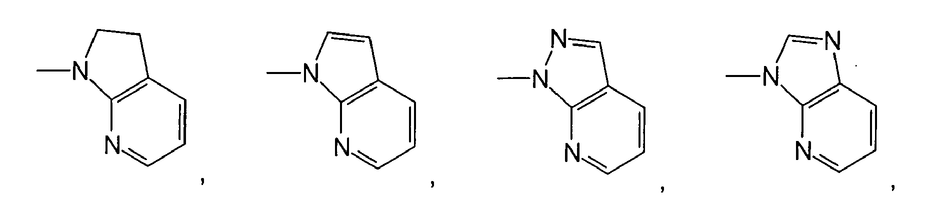 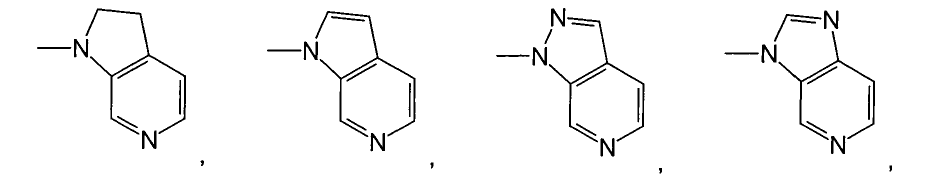 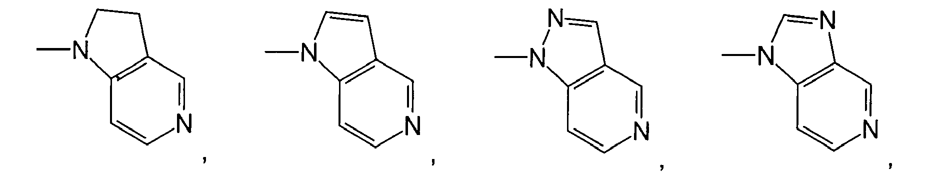 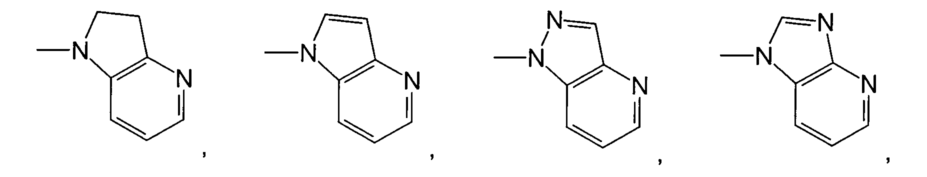 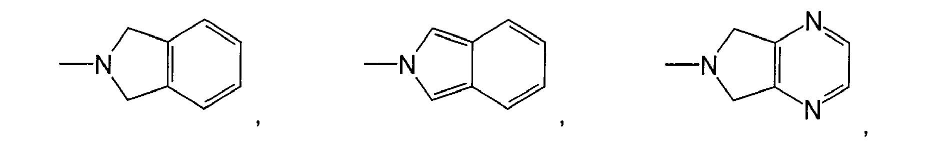 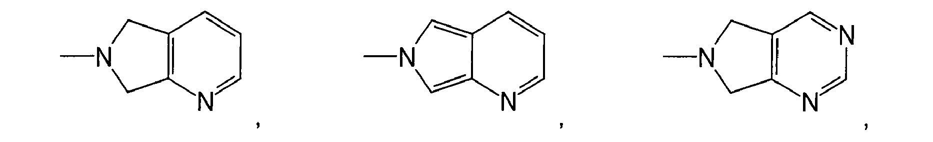 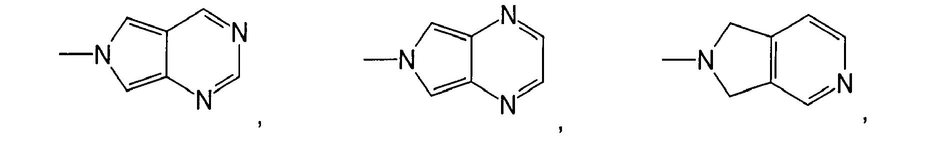 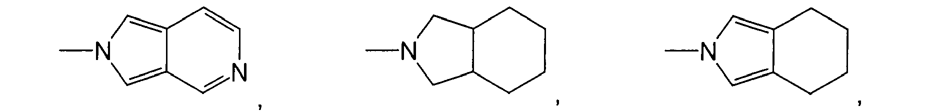 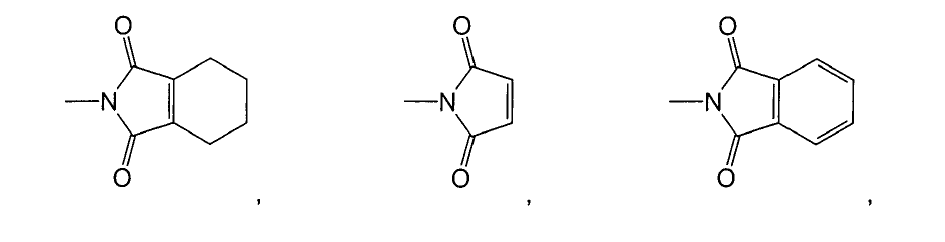 -A- and -A*- represent independently of each other:
-(CH₂)ₘ-, -(CH₂)ₙ-, -(CH₂)ₚ-(CH=CH)_{q}-(CH₂)ᵣ-, -(CH₂)ₛ-(C≡C)ₜ-(CH₂)ᵤ-, -(CH₂)ᵥ-(O-C₆H₄)_{w}-(CH₂)ₓ-, -(CH₂)ᵥ-(m-C₆H₄)_{w}-(CH₂)ₓ-, -(CH₂)ᵥ-(P-C₆H₄)_{w}-(CH₂)ₓ-,
**R⁴** and **R⁵** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇, -S-cyclo-C₃H₅, -SCH(CH₃)₂, -SC(CH₃)₃, -NO₂, -F, -Cl, -Br, -l, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃]_{,} -NH-CO-N(CH₃)_{2,} -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -CON(cyclo-C₃H₅)₂, -NH-C(=NH)-NH-cyclo-C₃H₅, -NH-C(=NH)-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NH₂, -NH-C(=NH)-N[CH(CH₃)₂]₂, -NH-C(=NH)-N[C(CH₃)₃]₂, -O-CO-O-cyclo-C₃H₅, -O-CO-NHCH₃, -O-CO-NHC₂H₅, -O-CO-NHC₃H₇, -O-CO-NH-cyclo-C₃H₅, -O-CO-NH[CH(CH₃)₂], -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-OCH(CH₃)₂, -O-CO-OC(CH₃)₃; 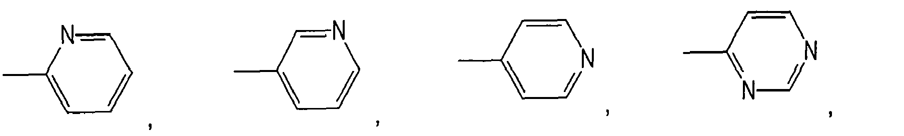 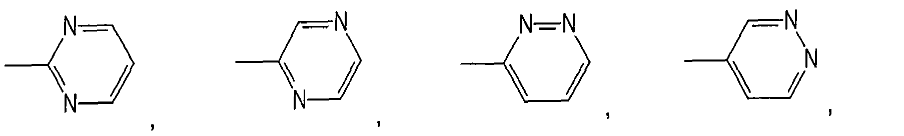 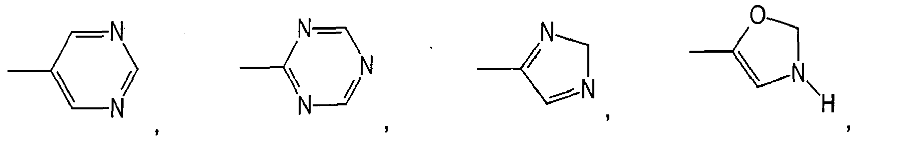 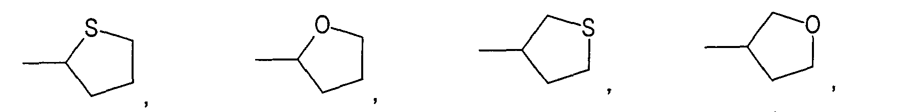 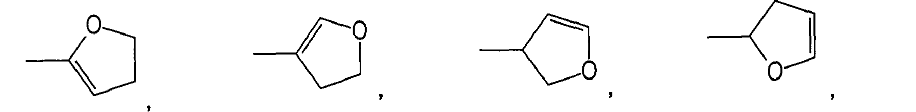 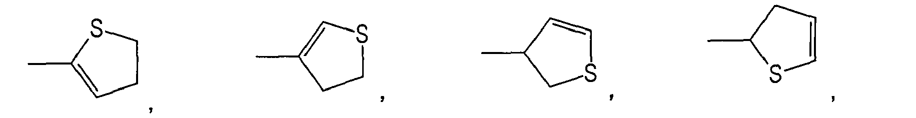 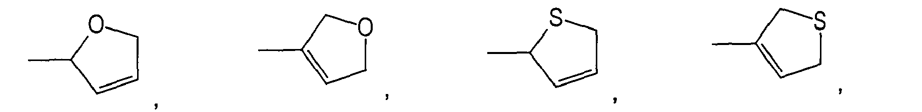 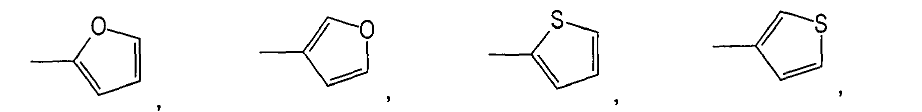 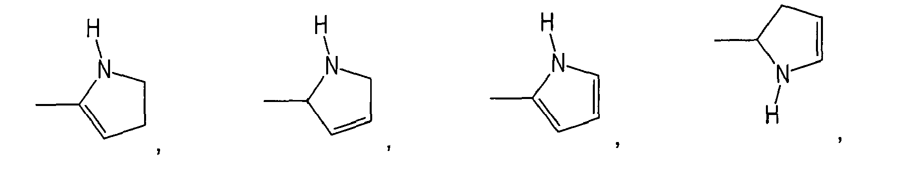 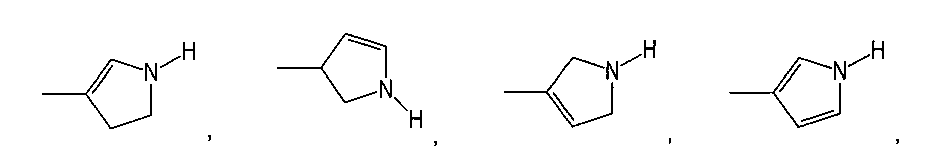 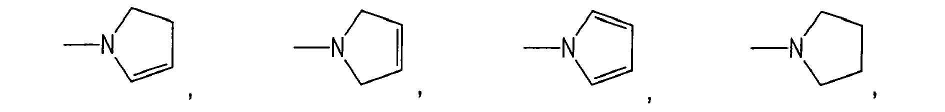 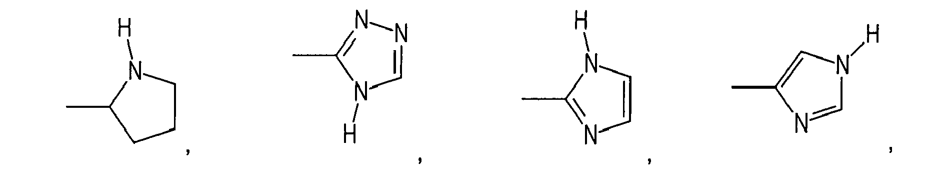 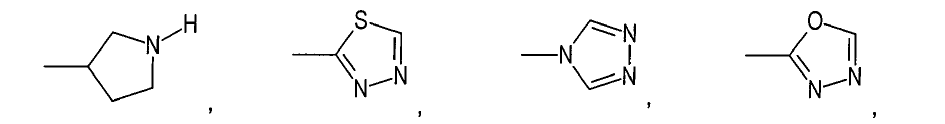 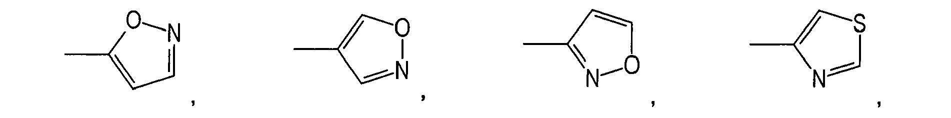 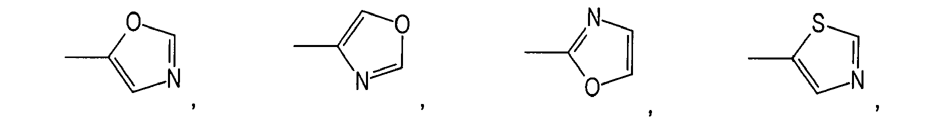 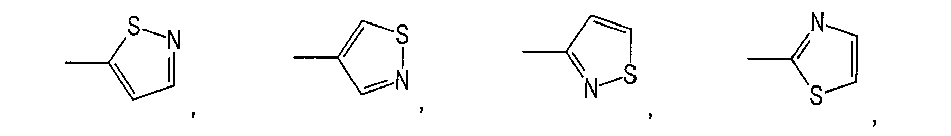 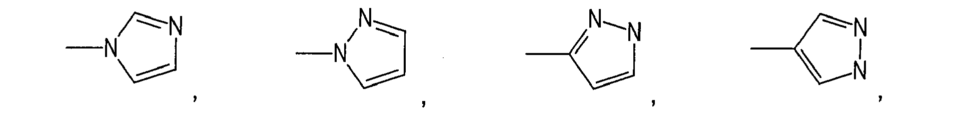 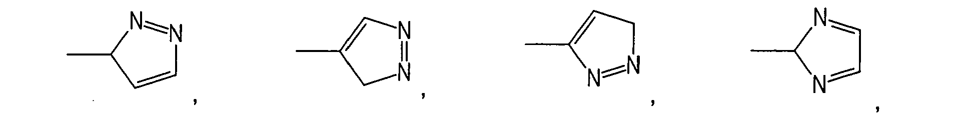 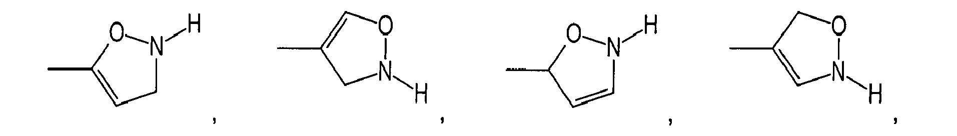 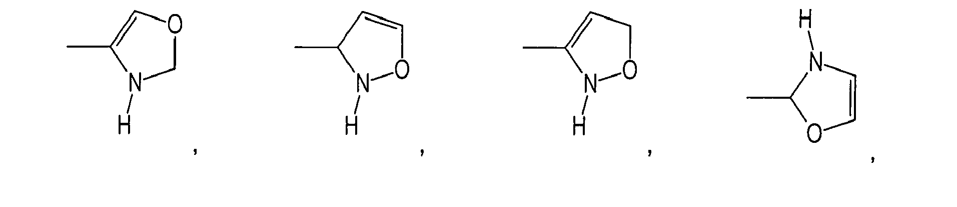 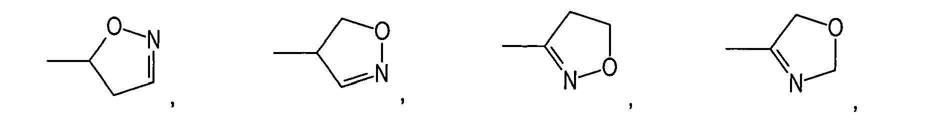 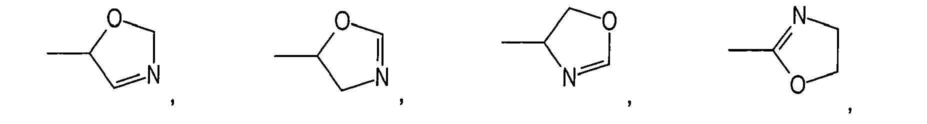 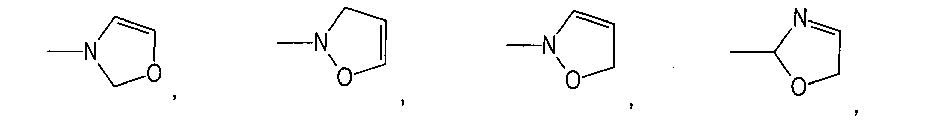 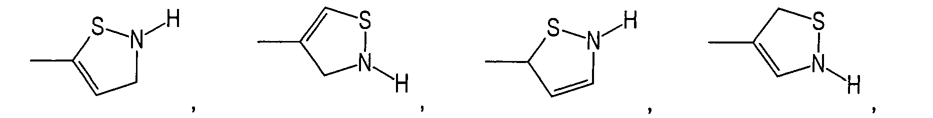 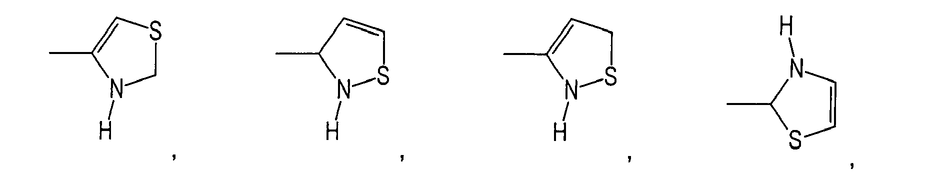 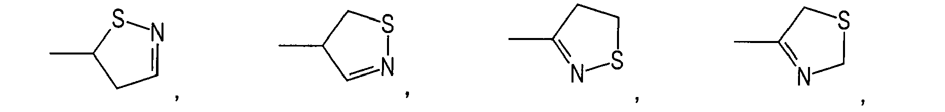 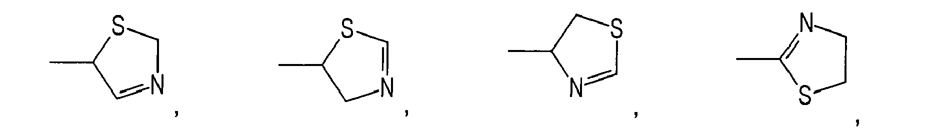 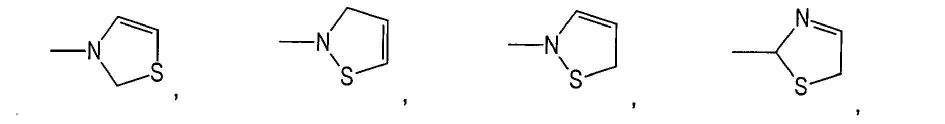 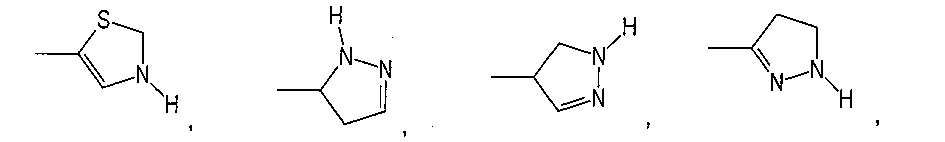 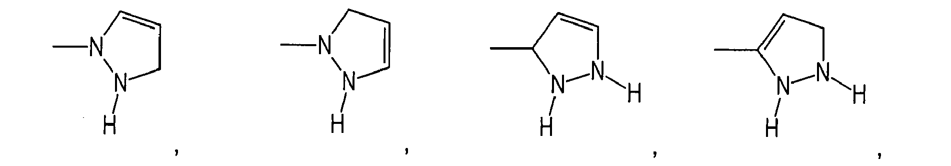 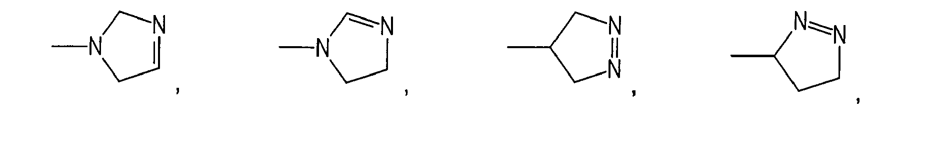 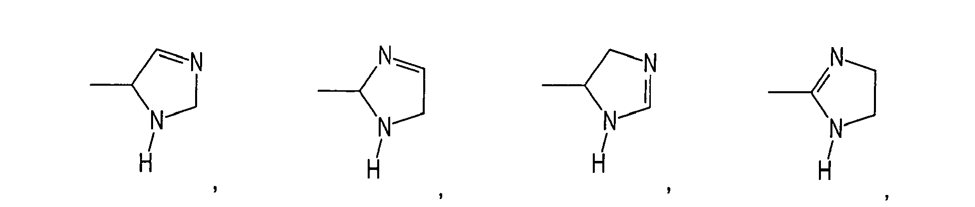 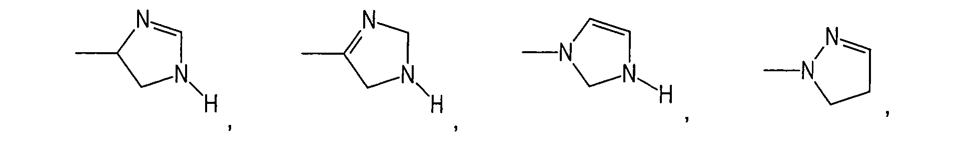 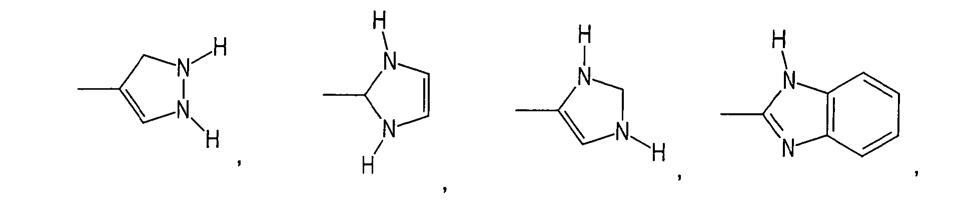 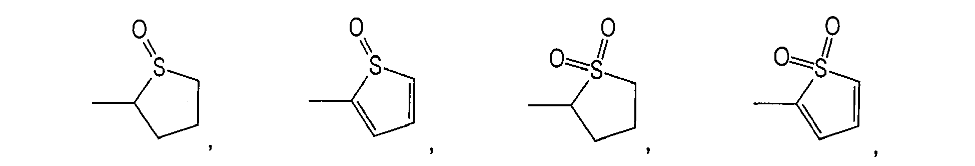 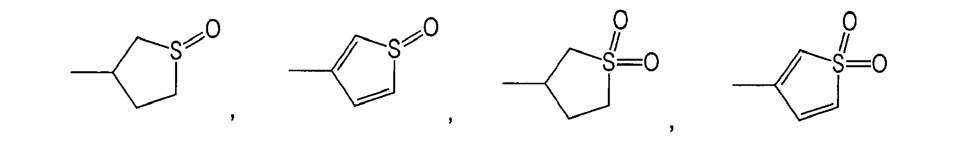 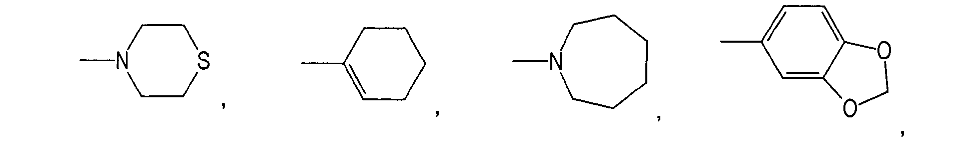 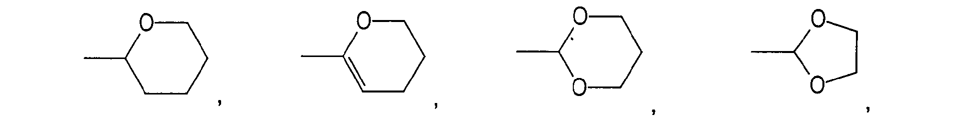 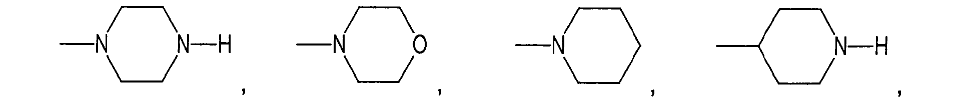 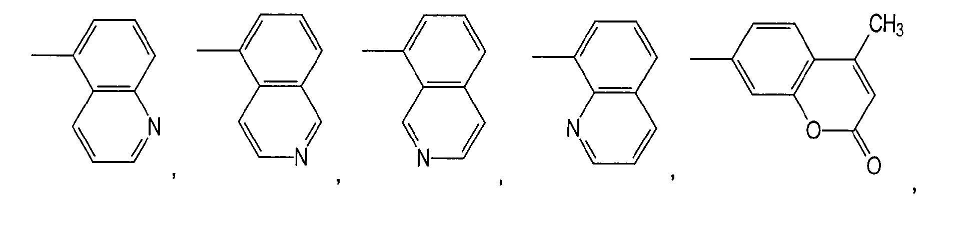 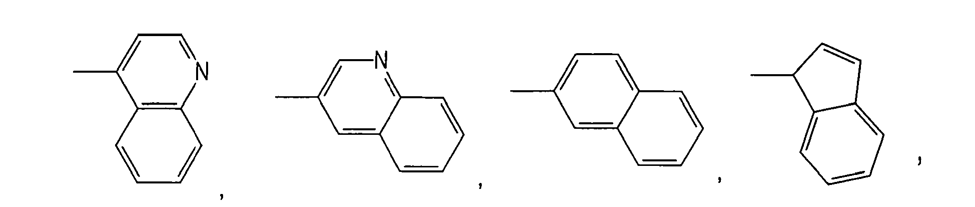 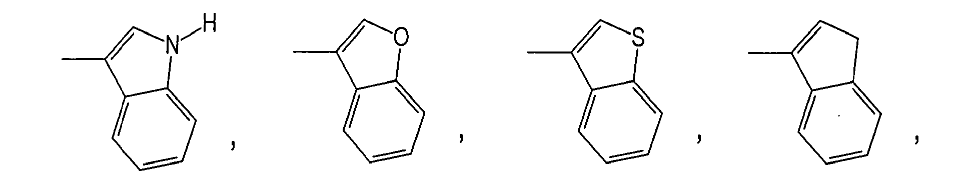 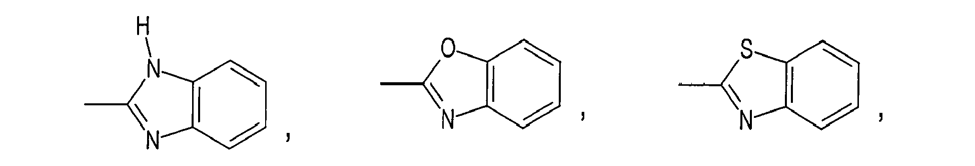 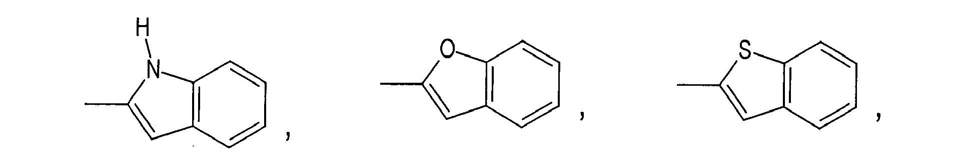 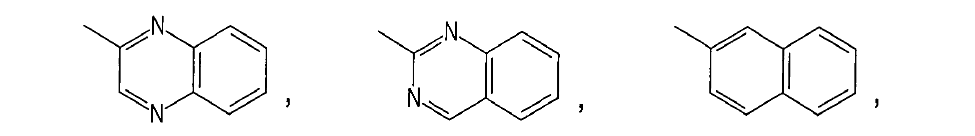 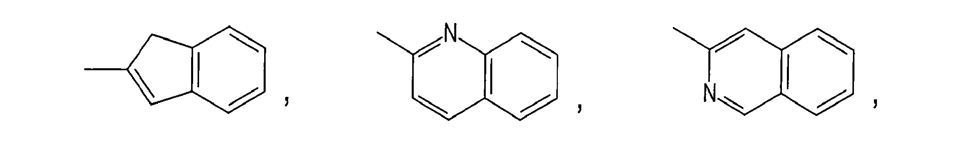 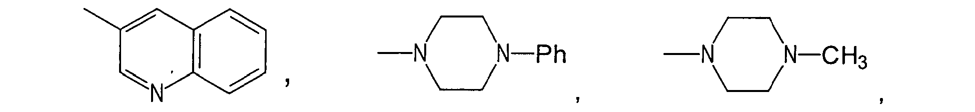 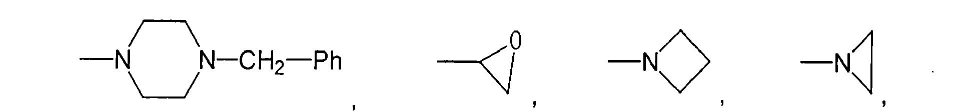 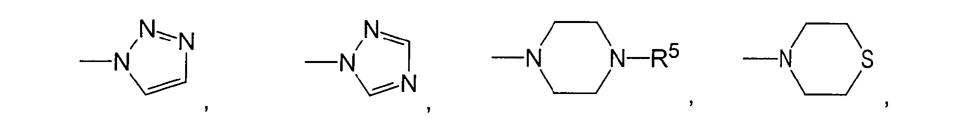 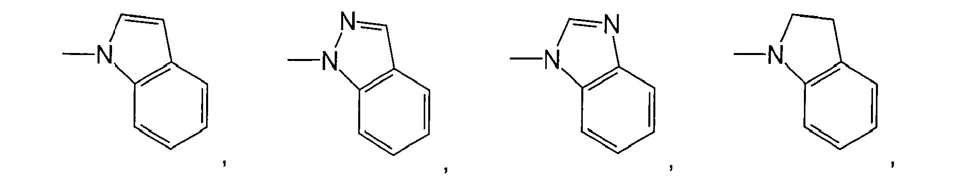 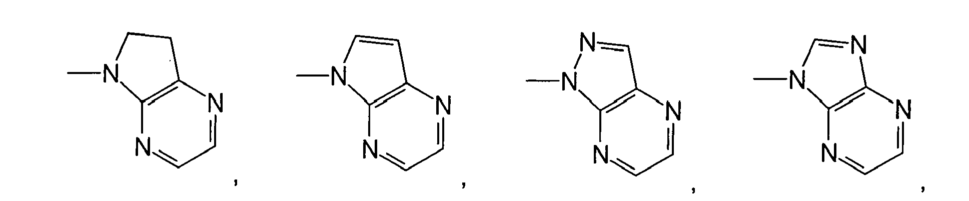 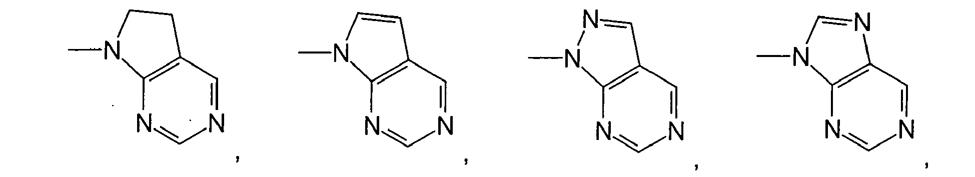 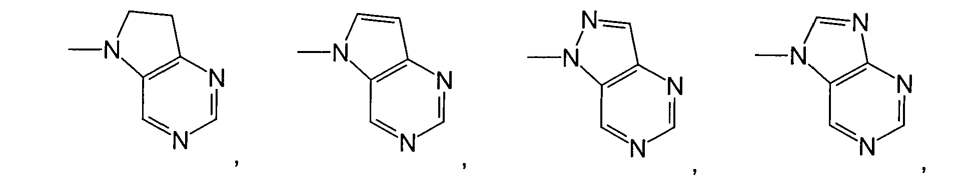 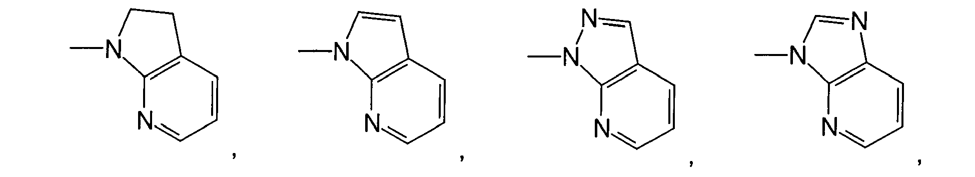 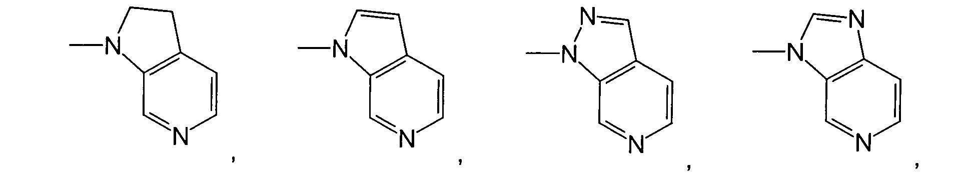 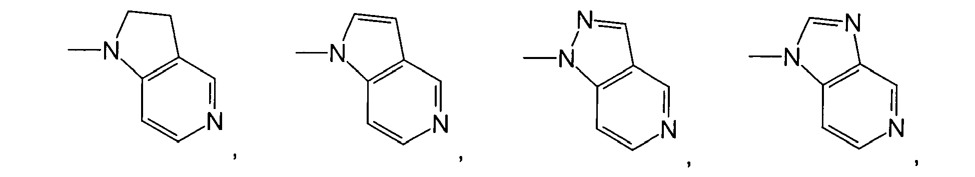 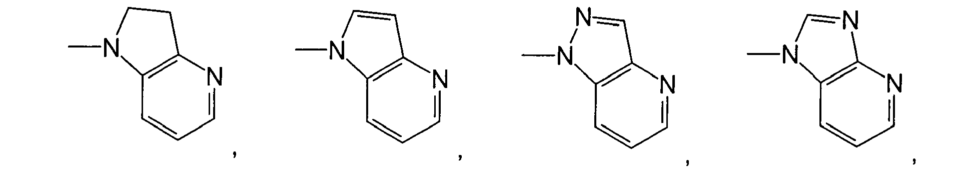 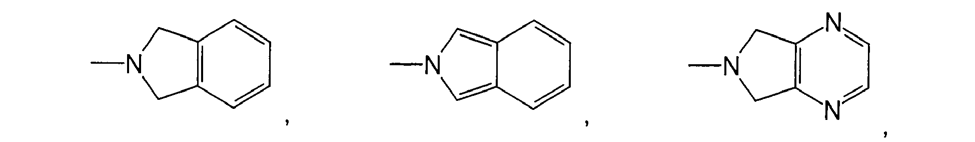 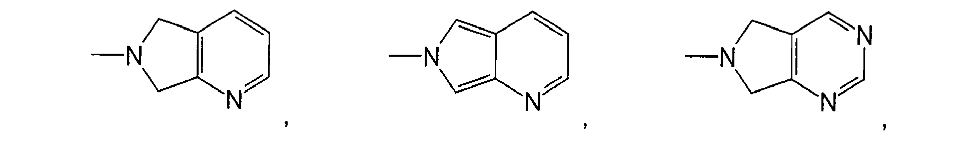 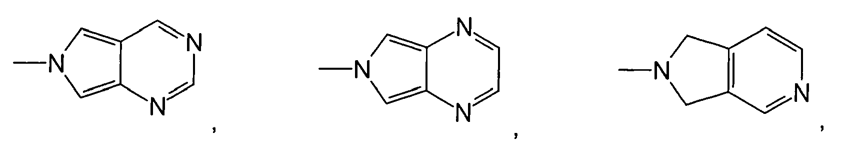 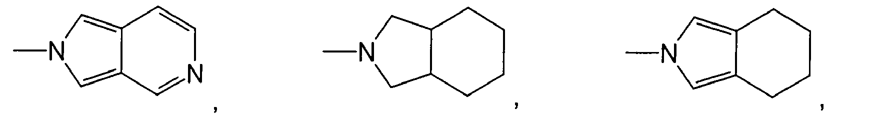 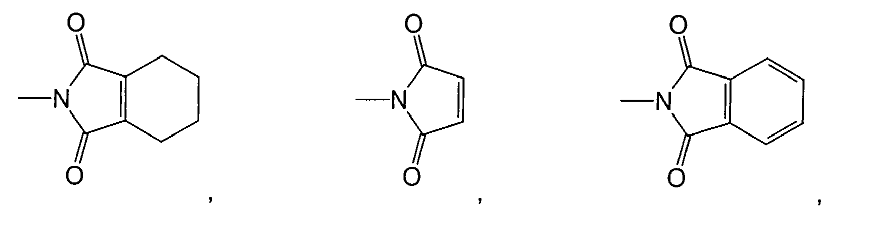
**R⁸** - **R¹⁶** represent independently of each other
-H, -CH₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, (CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆CH=CH-CH₃, -C₂H₄-CH=CH-C₂H₅, -CH₂-CH=CH-C₃H₇, -CH=CH-C₄H₉, -C₃H₆C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -CH₂-CH(C≡CH)₂, -C₂H₄-C(CH₃)=CH-CH₃, -CH₂-CH(CH₃)-CH=CH-CH₃, -C≡C-C≡CH, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-C≡C-C≡CH, -CH(CH₃)-CH₂-C(CH₃)=CH₂, -CH(CH₃)-CH(CH₃)-CH=CH₂, -CH₂-C≡C-C≡CH, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -C≡C-C≡C-CH₃, -CH=C(CH₃)-CH(CH₃)₂, -C(CH₃)=CH-CH(CH₃)₂, -CH(C≡CH)-C≡C-CH₃, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -CH(C≡CH)₂, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH=CH-Ph, -CH₂-CH=CH-C(CH₃)=CH₂, -CH₂-CH=C(CH₃)-CH=CH₂, -C≡C-C≡C-C₂H₅, -CH₂-C(CH₃)=CH-CH=CH₂, -CH(CH₃)-CH₂-C≡CH, -CH₂-C≡C-CH₂-C≡CH, -CH(CH₃)-CH=CH-CH=CH₂, -CH=CH-CH₂-C(CH₃)=CH₂, -CH(CH₃)-C≡C-CH₃,-CH=CH-CH(CH₃)-CH=CH₂, -CH=C(CH₃)-CH₂-CH=CH₂, -C≡C-C₂H₄-C≡CH, -C₂H₄-CH(CH₃)-C≡CH, -C(CH₃)=CH-CH₂-CH=CH₂, -C≡C-CH(CH₃)-C≡CH, -CH=CH-CH=C(CH₃)₂, -CH₂-CH(CH₃)-CH₂-C≡CH, -C(C≡CH)₂-CH₃, -CH=CH-C(CH₃)=CH-CH₃, -CH=C(CH₃)-CH=CH-CH₃, -CH(C≡CH)-CH₂-C≡CH, -CH₂-CH(CH₃)-C≡CH, -C(CH₃)=CH-CH=CH-CH₃, -C₂H₄-C≡C-C≡CH, -CH=C(CH₃)-C(CH₃)=CH₂, -C(CH₃)=CH-C(CH₃)=CH₂, -C≡C-CH₂C≡C-CH₃, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH(C₃H₇)-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, ₋C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂C≡C-C≡C-CH₃, -CH₂-C(CH₃)₂-C≡CH, -CH(CH₃)-CH(CH₃)-C≡CH, -C(CH₃)(C₂H₅)-C≡CH, -Ph, -CH₂-Ph, 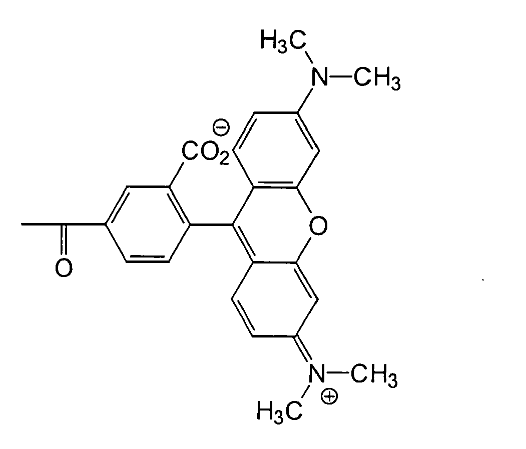
**k, I, m, n, p, r, s, t, u, v, w** and **x** represent independently of each other an integer 0, 1, 2, 3, 4, 5,6, 7, 8, 9, 10, 11, 12, 13, 14, 15 or 16;
and enantiomers, stereoisomeric forms, mixtures of enantiomers, diastereomers, mixtures of diastereomers, prodrugs, hydrates, solvates and racemates of the above mentioned compounds and pharmaceutically acceptable salts thereof.

2. Compound according to claim 1 having the general formula (B): 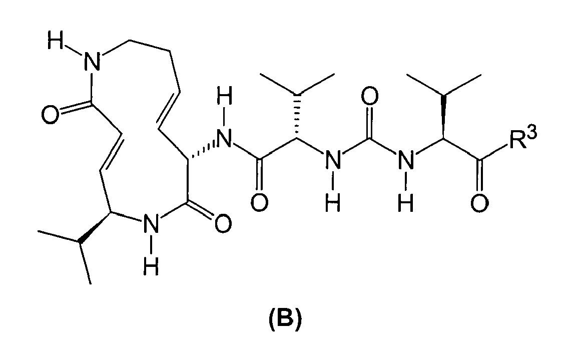 wherein
**R³** has the meanings as disclosed in claim 1.

3. Compound according to claim 1 having the general formula (C): 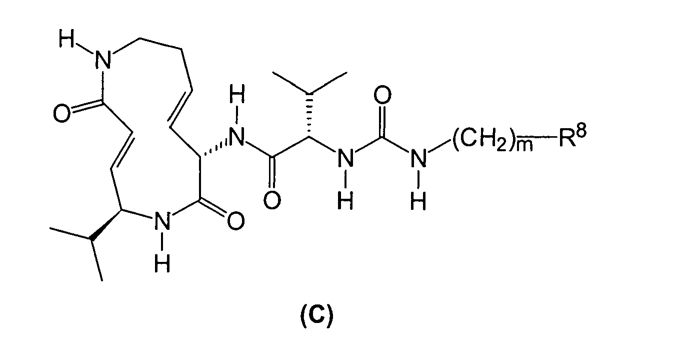 wherein **R⁸** and m have the meanings as disclosed in claim 1.

4. Compound according to claim 1 having the general formula (D): 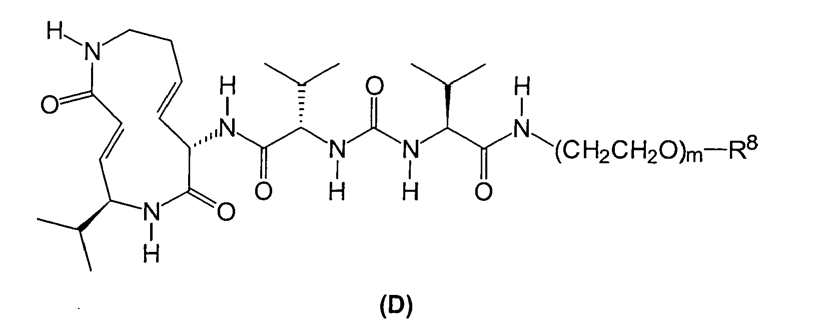 wherein **R⁸** and m have the meanings as disclosed in claim 1.

5. Compound according to claim 1 having the general formula (E): 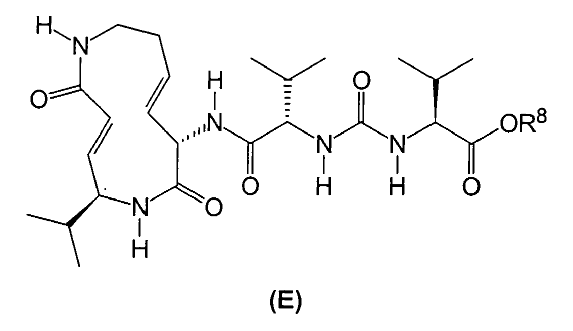 wherein **R⁸** has the meanings as disclosed in claim 1.

6. Compounds according to claim 1 selected from the group of:
SylA-OCH₃ (Compound 1),
SylA-Rhodamine (Compound 2),
SylA-PEG1 (Compound 3),
SylA-decanyl (Compound 4),
SylA-PEG2 (Compound 5).

7. Compounds according to any one of claims 1 - 6 for use as pharmaceutically active agent in medicine.

8. Compounds according to claim 7 for use as inhibitor of tau aggregation.

9. Use of a compound according to any one of claims 1 - 6 for the manufacture of a pharmaceutical composition for treatment or prophylaxis of neurodegenerative diseases, neurodegenerative disorders, neurodegenerative conditions, proliferative diseases, tumors, cancer, polyarthritis, psoriasis, allograft rejection, transplant rejection, graft-versus-host disease, cardiovascular diseases and cardiovascular endothelial disorders.

10. Use according to claim 9, wherein the neurodegenerative disease or neurodegenerative disorder or neurodegenerative condition is selected from the group comprising or consisting of: Alzheimer Disease, Parkinson Disease, Huntington Disease, amyotrophic lateral sclerosis, AIDS-related dementia, retinitis pigmentosa, spinal muscular atrophy and cerebrellar degeneration, fragile X-associated tremor/ataxia syndrome (FXTAS), progressive supranuclear palsy (PSP), and striatonigral degeneration (SND), which is included with olivopontocerebellear degeneration (OPCD), and Shy Drager syndrome (SDS) in a syndrome known as multiple system atrophy (MSA).

11. Use according to claim 9, wherein the proliferative disease or tumor or cancer is selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

12. Use according to claim 9, wherein the cardiovascular disease or cardiovascular endothelial disorder is selected from the group comprising or consisting of: adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, atherosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

13. Pharmaceutical composition comprising at least one compound according to any one of claims 1 - 6 as an active ingredient, together with at least one pharmaceutically acceptable carrier, excipient and/or diluents.

14. Pharmaceutical composition according to claim 13 further comprising at least one drug selected from the group comprising meclofenoxate, nicergoline, piracetame, pyritinole, tacrine, donezepiel, galantamine, rivastigmine, memantine, IMPase-inhibitors and extracts from Gingko biloba.

15. Method for synthesizing syringolin A derivatives comprising the steps:
reacting the amino compound 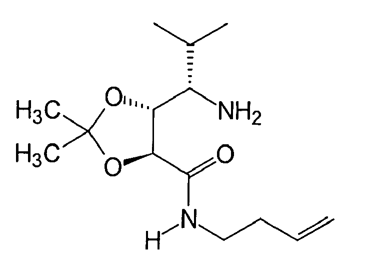 with the acid II 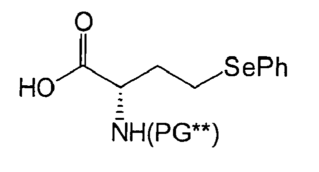 wherein PG** represents a suitable amino protecting group under conditions for forming an amide bond to obtain compound IV 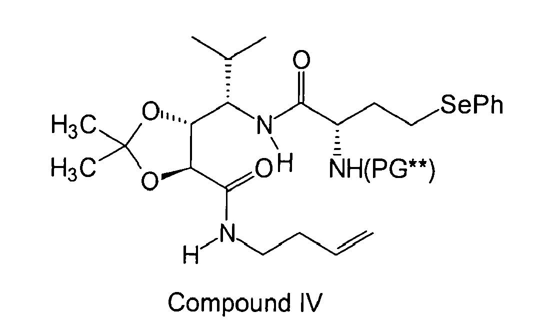
and oxidizing compound IV to compound V 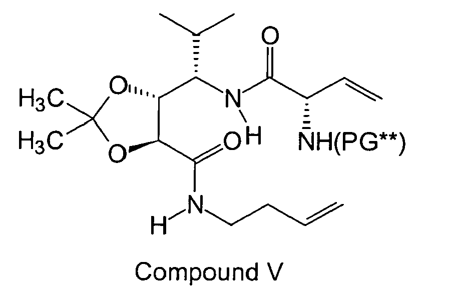 followed by a macrolactamisation step using Grubbs II catalyst to synthesize the macrocyclic compound VI 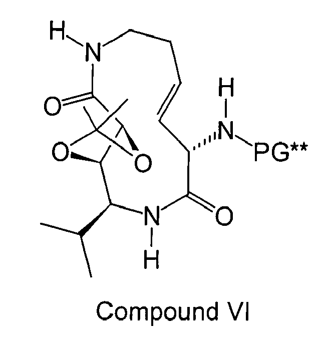 which ketal group is cleaved and converted to the thiocarbonate VII using (Im)₂CS 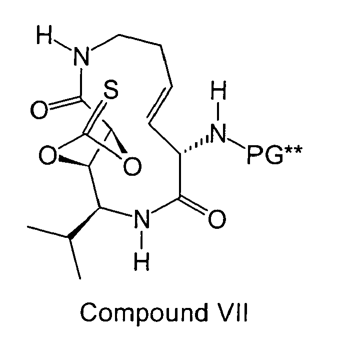 which is reduced with a phosphite to the double bond of compound VIII 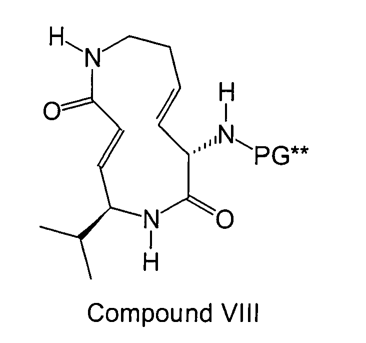 followed by a deprotection step of the amino group to compound IX 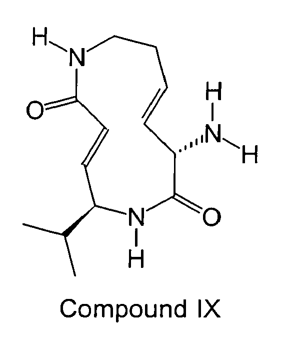 which is than reacted with the amino protected amino acid valin under common conditions for forming an amide bond in order to produce compound X 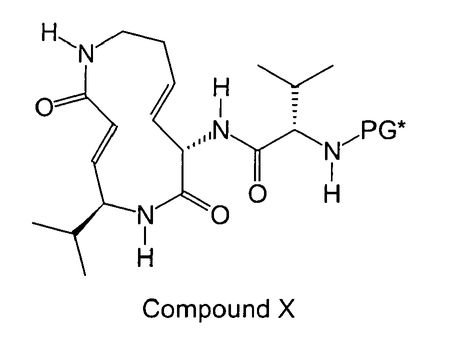 which is deprotected to obtain the key compound XI 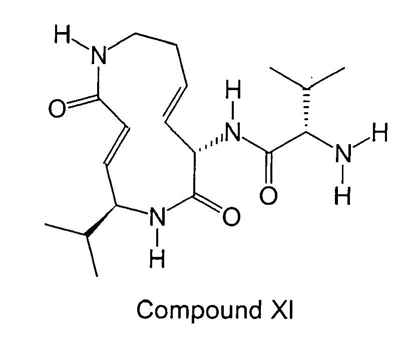 which is finally subjected to a triphosgene treatment which generates an intermediate chlorocarbonyl compound which is finally reacted with nucleophiles such as amines in order to obtain the final product of general formula A 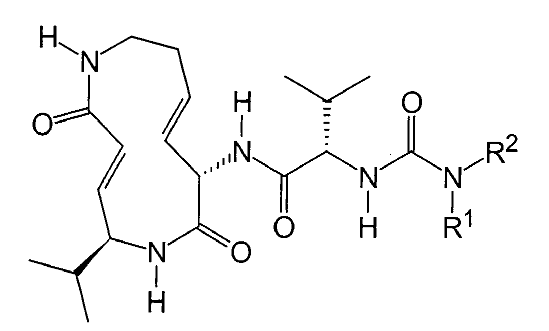 wherein
the substituents R¹ and R² have the meanings as disclosed in claim 1.
